(19) Europäisches Patentamt
European Patent Office
Office européen des brevets

(11) EP 2 223 996 B1

(12) # EUROPEAN PATENT SPECIFICATION

(45) Date of publication and mention of the grant of the patent:
**14.08.2013 Bulletin 2013/33**

(21) Application number: **08853809.5**

(22) Date of filing: **03.10.2008**

(51) Int Cl.:
***C12N 1/19*** (2006.01) ***C12N 1/15*** (2006.01)
***C12N 9/04*** (2006.01) ***C12N 15/09*** (2006.01)
***C12P 21/02*** (2006.01) ***C12Q 1/32*** (2006.01)
***C12Q 1/54*** (2006.01) ***C12Q 1/00*** (2006.01)

(86) International application number:
**PCT/JP2008/068011**

(87) International publication number:
**WO 2009/069381 (04.06.2009 Gazette 2009/23)**

(54) **TRANSFORMANT TRANSFECTED WITH FLAVIN ADENINE DINUCLEOTIDE-BINDING GLUCOSE DEHYDROGENASE GENE AND METHOD FOR PRODUCING FLAVIN ADENINE DINUCLEOTIDE-BINDING GLUCOSE DEHYDROGENASE USING THE SAME**

MIT DEM GEN FÜR FLAVIN-ADENIN-DINUKLEOTID BINDENDE GLUCOSE-DEHYDROGENASE TRANSFIZIERTE TRANSFORMANTE UND VERFAHREN ZUR HERSTELLUNG VON FLAVIN-ADENIN-DINUKLEOTID BINDENDER GLUCOSE-DEHYDROGENASE DAMIT

TRANSFORMANT TRANSFECTÉ PAR LE GÈNE DU GLUCOSE DÉSHYDROGÉNASE SE LIANT À LA FLAVINE ADÉNINE DINUCLÉOTIDE ET PROCÉDÉ DE FABRICATION DE GLUCOSE DÉSHYDROGÉNASE SE LIANT À LA FLAVINE ADÉNINE DINUCLÉOTIDE À L'AIDE DE CELUI-CI

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.11.2007 JP 2007308072**

(43) Date of publication of application:
**01.09.2010 Bulletin 2010/35**

(73) Proprietor: **Amano Enzyme Inc.**
**Nagoya-shi**
**Aichi 460-8630 (JP)**

(72) Inventors:
- **YUUKI, Kensuke**
**Kakamigahara-shi**
**Gifu 509-0109 (JP)**
- **NAKANISHI, Yuji**
**Kakamigahara-shi**
**Gifu 509-0109 (JP)**

(74) Representative: **Müller Fottner Steinecke**
**Rechtsanwälte Patentanwälte**
**Postfach 11 40**
**Römerstrasse 16 b**
**52412 Jülich (DE)**

(56) References cited:
**EP-A1- 2 022 850**
**WO-A1-2007/116710**
**WO-A1-2007/139013**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 223 996 B1

## Description

### TECHNICAL FIELD

The present invention relates to a transformant in which a flavin adenine dinucleotide-binding glucose dehydrogenase (also referred to as "FAD-GDH" in the present specification) gene has been introduced, and a method for producing FAD-GDH using the transformant.

### BACKGROUND ART

In recent years, a simplified self blood glucose meter using an electrochemical biosensor has been widely used. The biosensor has an electrode and an enzyme reaction layer formed on an insulating substrate. Examples of an enzyme used herein include glucose dehydrogenase (GDH) and glucose oxidase (GO). A method of using GO is easily affected by dissolved oxygen in a measurement sample, and such a problem that the dissolved oxygen gives an adverse effect on a measurement result has been pointed out.
On the other hand, as an enzyme that does not receive an effect of dissolved oxygen and acts on glucose in absence of NAD (P), GDH (PQQ-GDH) having pyrroloquinoline quinone (PQQ) as a coenzyme has been known (for example, see Patent Documents 1 to 3). However, PQQ-GDH has problems such that (1) PQQ is easily dissociated from an enzyme, (2) selectivity to glucose is low, and (3) since it is generally present in a membrane fraction, extraction and isolation operations thereof are performed with difficulty.
By the way, in recent years, a case of developing hypoglycemia, etc. in a patient who used a simplified self blood glucose meter in a hospital has been reported. As a result of an examination and analysis thereafter, it was revealed that such a developing case was caused by PQQ-GDH which was reacted to maltose contained as an infusion component and a higher value than an actual value was shown (Pharmaceuticals and Medical Devices Safety Information No. 206, October 2004, the Pharmaceutical and Medical Safety Bureau in the Ministry of Health, Labour and Welfare). In view of such a circumstance, development of a blood glucose meter that specifically acts on glucose and has low reactivity particularly to maltose has been eagerly desired.
In addition, as an enzyme that does not receive an effect of dissolved oxygen and acts on glucose in absence of NAD (P), other than PQQ-GDH, a glucose dehydrogenase having a flavin adenine dinucleotide as a coenzyme (that is, FAD-GDH) has been known. FAD-GDH has been obtained respectively from Aspergillus oryzae (Non-patent Documents 1 to 6) and Aspergillus terreus (Patent Document 4) so far. However, production amounts of the FAD-GDH are hardly recognized as sufficient production amounts supplied to industrial applications.

Patent Document 1: JP 2000-350588 A
Patent Document 2: JP 2001-197888 A
Patent Document 3: JP 2001-346587 A
Patent Document 4: WO No.2004/058958
Patent Document 5: EP 2 022 850 A
Patent Document 6: WO 2007/116710 A
Non-patent Document 1: Studies on the glucose dehydrogenase of Aspergillus oryzae. I. Induction of its synthesis by p-benzoquinone and hydroquinone, T.C. Bak, and R. Sato, Biochim. Biophys. Acta, 139, 265-276 (1967).
Non-patent Document 2: Studies on the glucose dehydrogenase of Aspergillus oryzae. II. Purification and physical and chemical properties, T.C. Bak, Biochim. Biophys. Acta, 139, 277-293 (1967).
Non-patent Document 3: Studies on the glucose dehydrogenase of Aspergillus oryzae. III. General enzymatic properties, T.C. Bak, Biochim. Biophys. Acta, 146, 317-327 (1967).
Non-patent Document 4: Studies on the glucose dehydrogenase of Aspergillus oryzae. IV. Histidyl residue as an active site, T.C. Bak, and R. Sato, Biochim. Biophys. Acta, 146, 328-335 (1967).

### DISCLOSURE OF INVENTION

### PROBLEMS TO BE SOLVED BY THE INVENTION

The study group of the present inventors focused on FAD-GDH having a flavin adenine dinucleotide as a coenzyme for the purpose of obtaining novel FAD-GDH capable of more accurately measuring a glucose level and a producing microorganism thereof. As a result of broadly screening microorganisms as objects, Aspergillus oryzae excellent in productivity of FAD-GDH was obtained. Further, they succeeded in purifying FAD-GDH that the fungus strain produced and also succeeded in determination of its various characteristics. The determined various characteristics revealed that the FAD-GDH is a novel enzyme. It was also revealed that the FAD-GDH is excellent in selectivity for glucose and at the same time is hardly affected from dissolved oxygen present in a reaction system, and thus is capable of more accurately measuring a glucose level in a sample. As a result of further progress of the studies, the present inventors succeeded in determining an amino acid sequence of FAD-GDH that the above described fungus strain has and a genetic sequence encoding the amino acid sequence. These results of the studies have been already filed in an international application (International Application No. PCT/JP2007/060694) corresponding to EP 2 022 850 A1.

In view of the above described backgrounds, an object of the present invention is to highly efficiently produce a large amount of novel FAD-GDH capable of more accurately measuring a glucose level.

Means for Solving the Problems

The present inventors made intensive studies in order to solve the above described problems. That is, they analyzed a gene encoding Aspergillus oryzae BB-56-derived FAD-GDH and found out that a specific portion of a base sequence gives effects on an optimal pH of a FAD-GDH enzyme, pH stability, temperature stability, substrate specificity, and FAD-GDH production ability of a microorganism, in other words, a functional aspect and productivity of the enzyme. As a result of producing a transformant of a microorganism on the basis of the above finding, the inventors succeeded in obtaining a transformant with extremely high productivity of FAD-GDH and thus achieved completion of the present invention. The present invention is stated as follows.

[1] A transformant, wherein the transformant is obtained by transforming *Aspergillus oryzae* and wherein the *Aspergillus oryzae* is a uridine requiring strain, in which a DNA encoding a flavin adenine dinucleotide-binding glucose dehydrogenase selected from the group consisting of the following (a) to (c) has been introduced:

(a) a DNA encoding the amino acid sequence of SEQ ID NO: 20;
(b) a DNA consisting of the base sequence of SEQ ID NO: 19;
(c) a DNA having a base sequence homologous to the base sequence of SEQ ID NO: 19 and encoding a protein having a flavin adenine dinucleotide-binding glucose dehydrogenase activity.

[2] The transformant according to [1], which is obtained by transforming a host microorganism belonging to the genus Aspergillus.
[3] The transformant according to [1], which is obtained by transforming Aspergillus oryzae.
For exemplary purpose is described herein a transformant in which a DNA encoding a flavin adenine dinucleotide-binding glucose dehydrogenase selected from the following group has been introduced:

a DNA encoding the amino acid sequence of SEQ ID NO: 34;
a DNA consisting of the base sequence of SEQ ID NO: 33; and
a DNA having a base sequence homologous to the base sequence of SEQ ID NO: 33 and encoding a protein having a flavin adenine dinucleotide-binding glucose dehydrogenase activity.

This transformant may be obtained by transforming Saccharomyces cerevisiae.
[4] The present invention further relates to a method for producing a flavin adenine dinucleotide-binding glucose dehydrogenase, including the following steps (1) and (2):

(1) a step of culturing the transformant according to any of [1] to [3] under the condition of producing a protein encoded by the DNA; and
(2) a step of recovering the produced protein.

The present invention can be used in a glucose measurement method, wherein glucose in a sample is measured using a flavin adenine dinucleotide-binding glucose dehydrogenase produced in the production method according to [4],
in a glucose measuring reagent, containing a flavin adenine dinucleotide-binding glucose dehydrogenase produced in the production method according to [5], or in
a glucose measuring kit, containing the glucose measuring reagent according to [7], which have been described for exemplary purposes.

BRIEF DESCRIPTION OF DRAWINGS

Fig. 1 shows a construction of pFG2.

Fig. 2 shows a construction flow of pFGP2.

Fig. 3 shows comparison of FAD-GDH activities in culture liquids.

Fig. 4 shows comparison of FAD-GDH production abilities among respective fungus strains belonging to Aspergillus oryzae. ND means a detection limit or less (No Detectable).

Fig. 5 shows an alignment view of DNA sequences encoding Aspergillus oryzae BB-56- and RIB40-derived FAD-GDH.

Fig. 6 shows an alignment view of amino acid sequences of Aspergillus oryzae BB-56- and RIB40-derived FAD-GDH.

Fig. 7 shows an alignment view of DNA sequences encoding FAD-GDH derived from 9 fungus strains belonging to Aspergillus oryzae.

Fig. 8 shows an alignment view of amino acid sequences encoding FAD-GDH derived from 9 fungus strains belonging to Aspergillus oryzae.

Fig. 9 shows comparisons of optimal pHs of FAD-GDH, Fig. 9(A) shows comparison of optimal pHs of Aspergillus oryzae BB-56-derived FAD-GDH, and Fig. 9(B) shows comparison of optimal pHs of Aspergillus oryzae RIB40-derived recombinant FAD-GDH.

Fig. 10 shows comparisons of pH stability of FAD-GDH, Fig. 10(A) shows comparison of pH stability of Aspergillus oryzae BB-56-derived FAD-GDH, Fig. 10(B) shows comparison of pH stability of Aspergillus oryzae RIB40-derived recombinant FAD-GDH.

Fig. 11 shows comparisons of temperature stability of FAD-GDH. Fig. 11 (A) shows comparison of temperature stability of Aspergillus oryzae BB-56-derived FAD-GDH, and Fig. 11(B) shows comparison of temperature stability of Aspergillus oryzae RIB40-derived recombinant FAD-GDH.

Fig. 12 shows comparison of substrate specificity of FAD-GDH. Numerical values in the table represent relative activities (%).

Fig. 13 shows comparison of FAD-GDH production abilities of transformants (yeasts). A FAD-GDH activity was not detected in both outside and inside of a fungus body from a yeast transformed with cDNA of RIB40-derived FAD-GDH.

Fig. 14 shows an elution result in SP Sepharose of FAD-GDH contained in a culture supernatant of a yeast transformed with cDNA encoding Aspergillus oryzae BB-56-derived FAD-GDH.

Fig. 15 shows an elution result in SP Sepharose of FAD-GDH contained in a culture supernatant of a yeast transformed with cDNA encoding Aspergillus oryzae RIB40-derived FAD-GDH.

Fig. 16 shows comparison between electrophoresis patterns of a SP Sepharose fraction and those after removal of sugar chains of wild-type Aspergillus oryzae RIB40-derived FAD-GDH. Lane 1: Aspergillus oryzae BB-56 purified FAD-GDH (non-treated), lane 2: Aspergillus oryzae BB-56 purified FAD-GDH (treated with endoglycosidase H), lane 3: Aspergillus oryzae BB-56-derived FAD-GDH (expressed with transformed yeast, non-treated), lane 4: Aspergillus oryzae BB-56-derived FAD-GDH (expressed with transformed yeast, treated with endoglycosidase H), lane 5: Aspergillus oryzae RIB40-derived FAD-GDH (expressed with transformed yeast, non-treated), and lane 6: Aspergillus oryzae RIB40-derived FAD-GDH (expressed with transformed yeast, treated with endoglycosidase H).

BEST MODE FOR CARRYING OUT THE INVENTION

(Terms)

A "DNA encoding a protein" in the present specification refers to a DNA capable of obtaining the protein when the DNA is expressed, that is, a DNA having a base sequence corresponding to an amino acid sequence of the protein. Therefore, degeneracy of codons is also taken into consideration.

The term "isolated" in the present specification is used exchangeably to "purified". "Isolated" when used in relation of the enzyme of the present invention (FAD-GDH), the term refers to a state in which, in the case where the enzyme of the present invention is derived from natural materials, components other than the enzyme are not substantially contained in the natural materials (in particular, contaminating protein is not substantially contained). Specifically, for example, in the isolated enzyme of the present invention, a content of contaminating protein is about less than 20% of the total, preferably less than 10%, more preferably less than 5%, and further more preferably less than 1% in weight conversion. On the other hand, the term "isolated" when the enzyme of the present invention is prepared in a genetic engineering technique refers to a state in which other components derived from a used host cell, a culture liquid, and the like are not substantially contained. Specifically, for example, in the isolated enzyme of the present invention, a content of contaminants is about less than 20% of the total, preferably less than 10%, more preferably less than 5%, and further more preferably less than 1% in weight conversion. In addition, as long as a different meaning from the above definition is not clearly specified, when "a flavin adenine dinucleotide-binding glucose dehydrogenase" simply described in the present specification, the wording means "a flavin adenine dinucleotide-binding glucose dehydrogenase in a state of being isolated". The terms of "FAD-GDH" and "enzyme" used in substitution for the flavin adenine dinucleotide-binding glucose dehydrogenase are also defined in the same manner.

"Isolated" as used for a DNA refers to a state in which, in the case of a DNA that is originally naturally present, typically, the DNA is separated from a nucleic acid that is coexistent in the natural condition. Provided that such a DNA may contain a part of other nucleic acid components such as an adjacent nucleic acid sequence in the natural condition (e.g., a sequence in a promoter region and a terminator sequence). For example, in the state of being "isolated" in the case of a genome DNA, other DNA components that are coexistent in the natural condition are not substantially contained. On the other hand, in the state of being "isolated" in the case of a DNA prepared in a genetic engineering technique, such as a cDNA molecule, the DNA preferably does not substantially contain cell components, a culture liquid, and the like. In the same manner, in the state of being "isolated" in the case of a DNA prepared through chemical synthesis, the DNA preferably does not substantially contain a precursor (raw material) such as dNTP and chemical substances used in synthetic processes. As long as a different meaning from the above definition is not clearly specified, when a "DNA" is simply described in the present specification, the term means a DNA in the state of being isolated.

(Transformant in which a DNA encoding FAD-GDH has been introduced)

The first aspect of the present invention provides a transformant in which a DNA encoding novel FAD-GDH has been introduced. In the transformant of the present invention, (a) a DNA encoding an amino acid sequence of SEQ ID NO: 20; (b) a DNA consisting of a base sequence of SEQ ID NO: 19; or (c) a DNA having a base sequence homologous to the base sequence of SEQ ID NO: 19 and encoding a protein having a flavin adenine dinucleotide-binding glucose dehydrogenase activity is present as an exogenous molecule. As an example, a further transformant has been described, wherein a DNA encoding an amino acid sequence of SEQ ID NO: 34; a DNA consisting of a base sequence of SEQ ID NO: 33; or a DNA having a base sequence homologous to the base sequence of SEQ ID NO: 33 and encoding a protein having a flavin adenine dinucleotide-binding glucose dehydrogenase activity is present as an exogenous molecule.

The amino acid sequence of SEQ ID NO: 20 is an amino acid sequence of FAD-GDH that Aspergillus oryzae BB-56 has, which was succeeded in identification by the present inventors. A specific example of a DNA encoding the amino acid sequence of SEQ ID NO: 20 is a DNA consisting of a base sequence of DNA SEQ ID NO: 19. Herein, when modification is performed on a part of a DNA encoding a certain protein, generally, a protein encoded by the DNA after modification may have functions equal to those of the protein encoded by the DNA before modification. That is, modification of a DNA sequence does not substantially give an effect on functions of a protein to be encoded and the functions of the protein to be encoded may be kept before and after modification. Thus, in one embodiment of the present invention, a DNA (c) having a base sequence homologous to the base sequence of SEQ ID NO: 19 and encoding a protein having a FAD-GDH activity is introduced. The "homologous base sequence" herein refers to a base sequence that is partly different from a nucleic acid of SEQ ID NO: 19 but is not substantially affected by the difference on the function (FAD-GDH activity is indicated herein) of a protein encoded by the nucleic acid.

A specific example of the homologous DNA includes a DNA that hybridizes under a stringent condition for a base sequence complementary to the base sequence of SEQ ID NO: 19. The "stringent condition" herein refers to a condition that a so-called specific hybrid is formed and a nonspecific hybrid was not formed. Such a stringent condition is known to a skilled person and can be set in reference to, for example, Molecular Cloning (Third Edition, Cold Spring Harbor Laboratory Press, New York) and Current protocols in molecular biology (edited by Frederick M. Ausubel et al., 1987). As the stringent condition, an example includes a condition such that a hybridization liquid (50% formamide, 10×SSC (0.15 M NaCl, 15 mM sodium citrate, pH 7.0), 5×Denhardt solution, 1% SDS, 10% dextran sulfate, 10 μg/ml of denatured salmon sperm DNA, and a 50 mM phosphate buffer (pH 7.5)) was used and incubated at about 42°C to about 50°C, and then washed at about 65°C to about 70°C, using 0.1×SSC, and 0.1 % SDS. An example of more preferable stringent condition is a condition of using 50% formamide, 5×SSC (0.15 M NaCl, 15 mM sodium citrate, pH 7.0), 1×Denhardt solution, 1% SDS, 10% dextran sulfate, 10 μg/ml of denatured salmon sperm DNA, and a 50 mM phosphate buffer (pH 7.5) as a hybridization liquid.

Other specific examples of the homologous DNA include a DNA consisting of a base sequence having the base sequence of SEQ ID NO: 19 as a base and containing substitution, deletion, insertion, addition or inversion of one base or plural bases, and encoding a protein having a FAD-GDH activity. Substitution, deletion, etc. of bases may be generated in plural sites. "Plural" herein refers to, for example, 2 to 40 bases, preferably 2 to 20 bases, and more preferably 2 to 10 bases, although it depends on a position or a kind of an amino acid residue in a cubic structure of a protein encoded by the DNA. Such a homologous DNA as described above can be obtained by modification of a DNA having the base sequence of SEQ ID NO: 19 so as to contain substitution, deletion, insertion, addition and/or inversion of bases, utilizing, for example, a restricted enzyme treatment, a treatment with exonuclease, DNA ligase, or the like, an introduction method of site-directed mutations (Molecular Cloning, Third Edition, Chapter 13, Cold Spring Harbor Laboratory Press, New York), an introduction method of random mutations (Molecular Cloning, Third Edition, Chapter 13, Cold Spring Harbor Laboratory Press, New York), or the like. In addition, a homologous DNA can also be obtained by other methods such as ultraviolet irradiation.

Other examples of the homologous DNA include a DNA in which difference of bases as described above is recognized due to polymorphism typically represented by SNP (single nucleotide polymorphism).

A DNA to be introduced for the transformant of the present invention can be prepared into a state of being isolated by use of a standard genetic engineering technique, molecular biological technique, biochemical technique, or the like, in reference to sequence information disclosed in the specification or attached sequence listing. Specifically, the DNA can be prepared by suitably using an oligonucleotide probe primer capable of hybridizing specifically to the DNA of the present invention from a genome DNA library or a cDNA library of suitable mould fungi or yeast fungi, or an extraction liquid of fungus bodies of mould fungi or yeast fungi. An oligonucleotide probe primer can be easily synthesized using a commercially available automation DNA synthesizer, or the like. For a production method of a library used to prepare the gene of the present invention, for example, Molecular Cloning, Third Edition, Cold Spring Harbor Laboratory Press, New York can be referred to.

When the DNA is a gene having the base sequence of SEQ ID NO: 19, for example, it can be isolated by use of a hybridization method in which whole or a part of the base sequence or its complementary sequence is used as a probe. Furthermore, the DNA can be amplified and isolated by use of a nucleic acid amplification reaction (for example, PCR) using a synthesized oligonucleotide primer designed so as to hybridize specifically to a part of the base sequence.

By the way, FAD-GDH that was succeeded in identification by the present inventors (an enzyme having an amino acid sequence of SEQ ID NO: 20, herein referred to as "the present enzyme") has the following properties (for details, see International Application No. PCT/JP2007/060694 described above). First, the present enzyme catalyzes a following reaction, that is, a reaction of generating glucono-$\delta$-lactone by oxidizing a hydroxyl group of glucose in the presence of an electron receptor. The molecular weight of the present enzyme by SDS-polyacrylamide gel electrophoresis is about 100 kDa, and the molecular weight of the present enzyme by gel filtration is about 400 kDa (tetramer).

On the other hand, the present enzyme is excellent in substrate specificity and selectively acts on D-glucose. Specifically, FAD-GDH of the present invention has an extremely low reactivity to maltose and also has a very low reactivity to D-fructose, D-mannose, D-galactose, and the like. Specifically, when a reactivity to D-glucose is assumed to be 100%, reactivities to these substrates are all 5% or less. In a preferable embodiment of the present invention, a reactivity to maltose is 1% or less or is not substantially recognized. In a more preferable embodiment, both of a reactivity to D-fructose and a reactivity to D-galactose are 1% or less or are not substantially recognized. The present enzyme having excellent substrate specificity as described above is preferable as an enzyme for accurately measuring a glucose level in a sample. That is, according to the present enzyme, even when impure substances such as maltose and galactose are present in the sample, a desired glucose level can be more accurately measured. Therefore, the present enzyme can be recognized as being suitable for an application (typically, measurement of a glucose level in blood) in which presence of such contaminants in a sample is expected or concerned, and also considered to be applicable to various applications including the application, that is, have high versatility.

As described above, the present enzyme was obtained from an Aspergillus oryzae BB-56 strain. Note that the fungus strain is deposited in an appointed deposit institution as described below and is easily available.

Deposit institution: Incorporated Administrative Agency, Patent Microorganisms Depositary, National Institute of Technology and Evaluation (2-5-8 Kazusakamatari, Kisarazu-shi, Chiba 292-0818, Japan)
Date of deposit: May 17, 2006
Accession number: NITE BP-236

It was revealed that the present enzyme obtained from Aspergillus oryzae BB-56 further has the following properties (for details, see International Application No. PCT/JP2007/060694 described above).

(4) Optimal pH: around 7
(5) Optimal temperature: around 60°C
(6) pH stability: stable within the range from pH 3.0 to 7.0
(7) Temperature stability: stable at 40°C or less
(8) Km value: Km value for D-glucose is about 8 mM
(9) Isoelectric point (pI): around 6.4

As described above, it is revealed that the present enzyme has selectivity and high affinity to glucose, is also excellent in stability, and suitable for applications and practical uses to a glucose sensor, and the like.

The transformant of the present inventor is typically prepared through transformation using a DNA to be introduced, or a DNA construct containing the DNA (e.g. a form of a vector). In the case of transformation with a vector, an expression vector is preferably used. The "expression vector" refers to a vector that can introduce a nucleic acid inserted therein into a desired cell (host cell) and is capable of expressing in the cell. The expression vector generally includes a promoter sequence necessary for expression of an inserted nucleic acid. Further, the expression vector preferably contains an enhancer sequence that promotes expression. An expression vector containing a selective marker can also be used. When such an expression vector is used, presence of absence (or its degree) of introduction of the expression vector can be confirmed utilizing a selective marker.

Insertion of the gene of the present invention into a vector, insertion of a selective marker gene (when required), insertion of a promoter (when required), and the like can be performed using a standard recombinant DNA technique (for example, a known method of using a restricted enzyme and DNA ligase, which can be referred to Molecular Cloning, Third Edition, 1.84, Cold Spring Harbor Laboratory Press, New York).

Transformation can be performed by calcium phosphate co-precipitation, electroporation (Potter, H. et al., Proc. Natl. Acad. Sci. U.S.A. 81, 7161-7165 (1984)), lipofection (Felgner, P.L. et al., Proc. Natl. Acad. Sci. U.S.A. 84,7413-7417 (1984)), Microinjection (Graessmann, M. & Graessmann, A., Proc. Natl. Acad. Sci. U.S.A. 73,366-370 (1976)), the Hanahan method (Hanahan, D., J. Mol. Biol. 166, 557-580 (1983)), the lithium acetate method (Schiestl, R.H. et al., Curr. Genet. 16, 339-346 (1989)), the protoplast-polyethylene glycol method (Yelton, M.M. et al., Proc. Natl. Acad. Sci. 81, 1470-1474 (1984)), or the like.

The host cell in the present invention is Aspergillus oryzae. However, in general, examples of a host cell include bacterial cells such as E. coli, mould fungi cells (e.g., Aspergillus oryzae, and Aspergillus niger), and yeast cells (e.g., Saccharomyces cerevisiae, Pichia pastoris, and Schizosaccharomyces pombe).

In general, microorganisms belonging to the genus Aspergillus or microorganisms belonging to the genus Saccharomyces may be used as a host. Examples of microorganisms belonging to the genus Aspergillus include Aspergillus oryzae, Aspergillus niger, Aspergillus nidulans, Aspergillus terreus, and Aspergillus awamori, and Aspergillus oryzae of a gene supply species that is considered to have a structure necessary for correct expression of an enzyme protein is the most preferable. On the other hand, examples of microorganisms belonging to the genus Saccharomyces include Saccharomyces cerevisiae, Pichia pastoris and Schizosaccharomyces pombe.

(Production method of FAD-GDH)

The second aspect of the present invention provides a method of producing FAD-GDH using the above described transformant. In the production method of the present invention, first, the transformant is cultured under the condition of producing a protein encoded with a gene introduced therein (step (1)). A culture method and culture conditions are not particularly limited as long as a desired enzyme is produced. That is, a method and culture conditions suitable for culture of a transformant in use can be appropriately set on condition of producing the present enzyme. In the following, a medium, a culture temperature, and a culture time are exemplified as the culture conditions.

As a medium, any medium may be used as long as it is a medium capable of growing a transformant in use. For example, carbon sources such as glucose, sucrose, gentiobiose, soluble starch, glycerin, dextrin, bees wax, and organic acids, further, ammonium sulfate, ammonium carbonate, ammonium phosphate, ammonium acetate, or nitrogen sources such as peptone, yeast extract, corn steep liquor, casein hydrolysate, bran, and meat extracts, and furthermore, substances added with inorganic salts such as potassium salt, magnesium salt, sodium salt, phosphate salt, manganese salt, iron salt, and zinc salt can be used. Vitamins, amino acid, and the like may be added to a medium in order to promote growth of the transformant in use. The pH of the medium is adjusted to, for example, about 3 to 8, and preferably about 5 to 7, and the transformant is cultured in an aerobic condition at a culture temperature of generally about 10 to 50°C, and preferably about 25 to 35°C, for 1 to 15 days, and preferably 3 to 7 days. As a culturing method, examples such as shake culture, aerobic submerged culture with a Jar fermentor can be used.

After the culture step, a produced protein (that is, FAD-GDH) is recovered (step (2)). When FAD-GDH can be recovered from the culture liquid, for example, the culture supernatant is treated by filtration and centrifugation to remove insoluble substances, and then, separation and purification are performed suitably combining with condensation with an ultrafilter membrane, salting out such as ammonium sulfate precipitation, dialysis, various chromatographies, and the like to thus obtain desired FAD-GDH. On the other hand, in the case of recovering from a fungus body, for example, a fungus body is crushed with a pressure treatment, an ultrasonic treatment, or the like, and then, separation and purification are performed in the same manner as described above to thus obtain desired FAD-GDH. In addition, the above described series of steps (fracture of fungus body, separation, and purification) may be performed after recovering a fungus body from a culture liquid previously by filtration, centrifugation, and the like.

Fractionation is performed using a FAD-GDH activity as an index as a general rule in each purification step, proceeding to the next step. However, the rule is not applied in the case that suitable conditions can be already set by a preliminary test, and the like.

Purity of FAD-GDH is not particularly limited and, for example, FAD-GDH can be purified so as to be in a state of having a relative activity of 50 to 160 (U/mg), and preferably 100 to 160 (U/mg). Further, FAD-GDH may be a liquid form or a solid form (including a powder state) in the final form.

(Application of FAD-GDH)

A further aspect described herein for exemplary reasons relates to an application use of FAD-GDH produced in the above described production method. First, a glucose measurement method using FAD-GDH is described for exemplary purposes. In the glucose measurement method, a glucose level in a sample is measured utilizing an oxidation-reduction reaction with FAD-GDH. The FAD-GDH produced is used in, for example, a measurement of a blood sugar level, a measurement of a glucose concentration in foods (such as seasonings and beverages), and the like. The produced FAD-GDH may be further used to examine a fermentation degree in production steps of fermented foods (e.g., vinegar) or fermented beverages (e.g., beer and alcohols). FAD-GDH is not required to be added with a coenzyme in measurement since FAD that is a coenzyme is constantly combined with GDH, and thus, a simple and easy measurement system can be constructed.

A reagent for glucose measurement may include FAD-GDH produced in the above described production method. The reagent is used in the above described glucose measurement method.

Furthermore, for exemplary reasons a kit for performing the glucose measurement method of the present invention (glucose measuring kit) is described herein. The kit includes a reaction reagent, a buffer solution, a glucose standard solution, and the like as optional elements, in addition to the reagent for glucose measurement containing FAD-GDH produced in the above described production method. In addition, an instruction manual is generally accompanied with the glucose measuring kit.

The present invention will be specifically described below in reference to examples. In the examples, products made by TAKARA BIO INC. and Toyobo Co., Ltd. were used as restricted enzymes and other genetic manipulation enzymes, as otherwise particularly stated. Note that reaction conditions, and the like of the enzymes were in accordance with attached instruction manuals. Determination of a base sequence was performed with an ABI PRISM™ 310 Genetic Analyzer and a BigDye Terminator v3.1 Cycle Sequencing Kit (Applied Biosystems Co.), and a PCR reaction was performed with a GeneAmp PCR System 9600 (Perkin Elmer Japan Co., Ltd.). These apparatuses, and the apparatuses and products for genetic manipulation used in the examples were operated in accordance with recommended conditions in instruction manuals attached to the respective products as otherwise particularly stated.

Example 1

<Culture of Aspergillus oryzae BB-56>

According to the following procedure, Aspergillus oryzae BB-56 was cultured to produce FAD-GDH. 50 ml of a medium for preliminary culture, which was constituted with the components below, was dividedly charged in a 300 mL-volume conical flask and sterilized at 121°C and 0.12 MPa for 20 minutes. The medium was cooled to room temperature, and Aspergillus oryzae BB-56 was then inoculated and cultured at 30°C and 200 rpm for 3 days.

(Medium components for preliminary culture)

Yeast extract (Becton, Dickinson Company) 0.2% (w/v)
Soybean peptone (DMV Co.) 1.0% (w/v)
Glucose (Wako Pure Chemical Industries, Ltd.) 2.0% (w/v)
$KH_2PO_4$ (Wako Pure Chemical Industries, Ltd.) 0.1% (w/v)
$MgSO_4 \cdot 7H_2O$ (Sigma-Aldrich Japan K.K.) 0.05% (w/v)
Medium pH 5.7

FAD-GDH was produced with a medium for main culture constituted with the components below. 50 ml of the medium for main culture was dividedly charged in a 300 mL-volume conical flask and sterilized at 121°C and 0.12 MPa for 20 minutes. The medium was cooled to room temperature, 1 mL of the culture liquid of the above preliminary culture was then inoculated, and the main culture was performed at 30°C and 200 rpm for 4 days to produce FAD-GDH.

(Medium components for main culture)

Glucose 15.0% (w/v)
Meast P1G (ASAHI FOOD & HEALTHCARE CO., LTD.) 3.0% (w/v)

Soybean peptone 6.0% (w/v)
$KH_2PO_4$ 0.3% (w/v)
$K_2HPO_4$ (Wako Pure Chemical Industries, Ltd.) 0.2% (w/v)
4 mM Hydroquinone (Wako Pure Chemical Industries, Ltd.)
Medium pH 6.0

Example 2

<Purification of Aspergillus oryzae BB-56-derived FAD-GDH>

FAD-GDH was purified under the following conditions. Fungus bodies and other solid contents in the culture liquid were removed by diatomite filtration of the culture liquid obtained as described above. The purified liquid obtained through the operation was desalinated and concentrated with an ultrafilter membrane. The concentrated liquid was subjected to salting out with 90% saturated ammonium sulfate and the centrifuged supernatant was desalinated and concentrated with an ultrafilter membrane. The desalinated and concentrated liquid was applied to a CM Sepharose Fast Flow (Amersham Biosciences Co.) equilibrated with a 10 mM McIlvaine buffer solution at pH 5.5 to adsorb FAD-GDH to a column. The column was washed with a 10 mM McIlvaine buffer solution at pH 5.5 and FAD-GDH was eluted with a 10 mM McIlvaine buffer solution containing 0.1 M of NaCl at pH 5.5 to recover a FAD-GDH active fraction. The recovered active fraction was concentrated with an ultrafilter membrane and applied to Octyl-Sepharose equilibrated with a 10 mM $K_2HPO_4$-NaOH buffer solution containing 2.5 M of ammonium sulfate at pH 6.5 to adsorb FAD-GDH to a column. The column was washed with a 10 mM $K_2HPO_4$-NaOH buffer solution containing 2.5 M of ammonium sulfate and FAD-GDH was then eluted by changing a concentration of ammonium sulfate in the buffer solution to 2.0, 1.5, 1.0 and 0.5 M in stages. The active fraction of FAD-GDH was recovered, and desalinated and concentrated with an ultrafilter membrane. The desalinated and concentrated liquid was applied to DEAE Sepharose™ CL-6B equilibrated with a 20 mM $K_2HPO_4$-NaOH buffer solution at pH 6.5 and FAD-GDH was eluted with a 20 mM $K_2HPO_4$-NaOH buffer solution at pH 6.5. This fraction was desalinated and concentrated with an ultrafilter membrane to thus obtain a purified enzyme preparation of FAD-GDH.

Example 3

<Identification of partial amino acid sequence of Aspergillus oryzae BB-56-derived FAD-GDH>

A partial amino acid sequence of Aspergillus oryzae BB-56-derived FAD-GDH was identified as follows.

(1) Isoelectric fractionation of FAD-GDH

3 mL of the purified FAD-GDH solution obtained in Example 2 was charged in a dialysis tube and immersed in purified water to perform dialysis at 4°C for one night. This dialyzed sample was supplied to glycerol density gradient gel electrophoresis. Energization was performed at 5°C at a low voltage of 400 V for 48 hours with a column volume of 110 mL using a carrier ampholyte (Pharmalyte 3-10 for IEF (Amersham Biosciences Co.)). After energization, the sample was recovered and a pH and a FAD-GDH activity of each fraction were measured to collect an active fraction.

(2) Preparation of sample for partial amino acid sequence analysis

The purified FAD-GDH obtained in the above described (1) was supplied to SDS-PAGE using a gel, PAG Mini "DAIICHI" 7.5 (Daiichi Pure Chemicals Co., Ltd.). Using a transcription buffer solution 1 (0.3 M tris (pH 10.4), 20% methanol), a transcription buffer solution 2 (30 mM tris (pH 10.4), 20% methanol), and a transcription buffer solution 3 (40 mM 6-aminohexanoic acid (pH 7.6), 20% methanol) as transcription buffer solutions, the gel after migration was transcribed to a PVDF membrane. The transcribed membrane after transcription was stained with Coomassie Brilliant Blue R-250 to detect and cut out a band corresponding to FAD-GDH. The cut out membrane was used as an analysis sample of an N-terminated amino acid sequence. In the same manner, the gel after SDS-PAGE was stained with Coomassie Brilliant Blue R250 to detect and cut out a band corresponding to FAD-GDH. The cut out gel was used as an analysis sample of an internal amino acid sequence. As a result of the analyses, the N-terminated amino acid sequence and the internal amino acid sequence were found out.

Example 4

<Homology search>

Homology in N-terminated amino acid sequences were searched as described below. All CDS sequences among the genetic information of Aspergillus oryzae RIB40 disclosed in JP 2005-176602 A were obtained from applicants of JP 2005-176602 A, homology search with N-terminated amino acid sequences found out in Example 3 was performed using database software, Kiroku Ver. 3 (World Fusion Co., Ltd.). As a result, high homology was recognized with an amino acid sequence (SEQ ID NO: 20494 in the sequence listing in JP 2005-176602 A) which has homology to a glucose oxidase precursor. In this sequence (SEQ ID NO: 20494 in the sequence listing in JP 2005-176602 A), a region corresponding to an analysis of an internal amino acid sequence of FAD-GDH identified in Example 3 was also present. From these facts, it was revealed that SEQ ID NO: 20494 (SEQ ID NO: 1) in the sequence listing in JP 2005-176602 A corresponds to an amino acid sequence of FAD-GDH.

Example 5

<Extraction of chromosome DNA derived from Aspergillus oryzae BB-56>

Aspergillus oryzae BB-56 was cultured at 30°C for one night using a shaking flask containing 100 ml of a YPD medium (1% yeast extract, 2% peptone, 2% glucose, pH 6.5), and then, the culture liquid was filtrated to obtain a fungus body. The fungus body was frozen and dried, and about 0.3 g of the dried fungus body was crushed with one spoonful of a scoopula of sea sand using a mortar, and suspended in 12 ml of an extraction buffer (1% hexadecyltrimethylammonium bromide, 0.7 M NaCl, 50 mM Tris-HCl (pH 8.0), 10 mM EDTA, 1% β-mercaptoethanol). After rotation stirring at room temperature for 30 minutes, an equivalent amount of a solution of phenol:chloroform:isoamyl alcohol (25:24:1, v/v) was added thereto and stirred, and thereafter centrifugation (1,500 g, 5 minutes, room temperature) was performed to obtain a supernatant. An equivalent amount of a solution of chloroform:isoamyl alcohol (24:1, v/v) was added to this supernatant and stirred, and thereafter centrifugation (1,500 g, 5 minutes, room temperature) was performed to obtain a supernatant. An equivalent amount of isopropanol was gently added to this supernatant, and centrifugation (20,000 g, 10 minutes, 4°C) was performed. The obtained precipitate (deposited chromosome DNA) was washed with 70% ethanol and vacuum dried to obtain a chromosome DNA. The chromosome DNA was dissolved in 4 ml of TE (composition), thereto was added 200 μl of 10 mg/ml RNase A (Sigma-Aldrich Japan K.K.), and the mixture was incubated at 37°C for 30 minutes. Then, 40 μl of a solution of 20 mg/ml Proteinase K, recombinant, PCR Grade (Roche Diagnostics K.K.) was added and incubated at 37°C for 30 minutes, and an equivalent amount of a solution of phenol:chloroform:isoamyl alcohol (25:24:1, v/v) was then added. After stirring, the resultant solution was centrifuged (1,500 g, 5 minutes, room temperature) to obtain a supernatant. This washing operation was performed twice, then, an equivalent amount of a solution of chloroform:isoamyl alcohol (24:1, v/v) was added to the supernatant and stirred, and centrifugation (1,500 g, 5 minutes, room temperature) was performed. To the obtained supernatant, 1/10 volume of 3M NaOAc (pH 4.8) and twice the volume of ethanol were added, and a chromosome DNA was deposited at -80°C. The deposited chromosome DNA was centrifuged (20,000 g, 20 minutes, 4°C) to thus recover as a precipitate. The recovered chromosome DNA was washed with 70% ethanol and then vacuum dried. The dried chromosome DNA was dissolved in 400 μl of a TE solution to thus obtain about 1 mg/ml of a chromosome DNA solution.

Example 6

<Design of synthesis primer>

Synthetic primers of FG-F and FG-R (SEQ ID NO: 3, 4) corresponding to a sequence of 5' and 3' ends of the Aspergillus oryzae RIB40-derived gene sequence (SEQ ID NO: 2) having the whole chain length of 3226 bp, which encodes the amino acid sequence (SEQ ID NO: 1) of SEQ ID NO: 20494 in the sequence listing of JP 2005-176602 A were synthesized.

Example 7

<Amplification of FAD-GDH gene in PCR method>

The Aspergillus oryzae BB-56-derived chromosome DNA prepared in Example 5 was used as a template, and a PCR reaction was performed. TAKARA LA Taq was used in the reaction, and the composition of the reaction solution was in accordance with a standard technique described in an attached instruction for use. Primers (FG-F, FG-R) were added in an each amount of 0.4 μM so that a template genome has a concentration of 10 ng/μl, and a PCR reaction was performed. The reaction cycle was as follows. After reacting at 94°C for 2 minutes, reactions of (i) to (iii) constituted with (i) a reaction at 94°C for 30 seconds, (ii) a reaction at 60°C for 30 seconds, and (iii) a reaction at 72°C for 3.5 minutes were repeated 35 times, and lastly, a reaction at 72°C for 10 minute was performed. After completion of the reactions, the reaction solution was preserved at 4°C.

Example 8

<Analysis of base sequence of Aspergillus oryzae BB-56-derived FAD-GDH gene>

The PCR reaction solution of Example 7 was supplied to agarose electrophoresis, and an amplified fragment (3241 bp) and primers were separated to be extracted from an agarose gel using a GENECLEAN III Kit (Q-BIOgene Co.). The extracted amplified fragment was sub-cloned to a *Sma*I site of a vector pUC 19 (TAKARA BIO INC.) to obtain pFG2 (Fig. 1). A PCR amplified fragment in pFG2, that is, a base sequence of an Aspergillus oryzae BB-56-derived FAD-GDH gene was analyzed. In order to analyze the base sequence of the above described gene, synthesis primers of FG-1 (SEQ ID NO: 5), FG-2 (SEQ ID NO: 6), FG-3 (SEQ ID NO: 7), FG-4(SEQ ID NO: 8), FG-5 (SEQ ID NO: 9), FG-6 (SEQ ID NO: 10), FG-7 (SEQ ID NO: 11), FG-8 (SEQ ID NO: 12), FG-9 (SEQ ID NO: 13), FG-10 (SEQ ID NO: 14), FG-11 (SEQ ID NO: 15), FG-12 (SEQ ID NO: 16), M13-M5 (SEQ ID NO: 17), and M13-RV2 (SEQ ID NO: 18) were formed. As a result of the analysis of the base sequence, the base sequence (SEQ ID NO: 19) of an Aspergillus oryzae BB-56-derived FAD-GDH gene having a whole chain length of 3241 bp was revealed. An amino acid sequence deduced from the gene sequence was represented by SEQ ID NO: 20.

Example 9

<Acquisition of high producing strain of Aspergillus oryzae BB-56-derived FAD-GDH >

A high producing strain of Aspergillus oryzae BB-56-derived FAD-GDH was obtained as follows.

(1) Amplification of orotidylate decarboxylase (pyrG) gene in PCR method

Sequence information of a pyrG gene of Aspergillus oryzae RIB40 was obtained from JP 2005-176602 A, and synthesis primers of PYR-F and PYR-R (SEQ ID NO: 21, 22) corresponding to 5' and 3' ends of the sequence were formed. A PCR reaction was performed using the chromosome DNA derived from Aspergillus oryzae BB-56 prepared in Example 5 as a template. TAKARA LA Taq was used in the reaction, and the composition of the reaction was in accordance with a standard technique described in an attached instruction for use. The reaction solution was prepared so that the primers of PYR-F and PYR-R were contained in an each amount of 0.4 μM and a chromosome DNA of the template has a concentration of 10 ng/μl, and the PCR reaction was performed. In the PCR reaction condition, after reacting at 94°C for 2 minutes, reactions of (i) to (iii) constituted with (i) a reaction at 94°C for 30 seconds, (ii) a reaction at 58°C for 30 seconds, and (iii) a reaction at 72°C for 2 minutes were repeated 35 times, and lastly, a reaction at 72°C for 10 minute was performed. After completion of the reactions, the reaction solution was preserved at 4°C.

(2) Construction of expression vector

The pyrG gene fragments (1884 bp) amplified by supplying the above described PCR reaction solution to agarose electrophoresis and the primers were separated and extracted from an agarose gel using a GENECLEAN III Kit (Q-BIOgene Co.). Both ends of the extracted pyrG gene fragment were digested with restricted enzymes, *Sal*I and *Hind*III. The DNA fragments treated with the restricted enzymes and pFG2 treated with *Sal*I and *Hind*III were ligated using a DNA Ligation Kit Ver. 2.1 to produce pFGP2 (Fig. 2). pFGP2 was introduced in an E. coli DH5 competent cell to thus obtain a transformant of E. coli.

(3) Preparation of gene fragment

The above described transformant of E. coli was cultured and a plasmid pFGP2 was extracted from the fungus body. The extracted pFGP2 was digested with restricted enzymes, *Hpa*I and *Hind*III, then supplied to agarose electrophoresis to separate about 5 kbp of a DNA fragment, and extracted from an agarose gel using a GENECLEAN III Kit (Q-BIOgene Co.). As expected, it was confirmed that 5 kbp of the DNA fragment was a DNA fragment formed by linking an Aspergillus oryzae BB-56-derived FAD-GDH gene and pyrG.

(4) Acquisition of Aspergillus oryzae BB-56-derived uridine requiring strain

Aspergillus oryzae BB-56 was cultured in a YPD medium (1% yeast extract, 2% peptone, 2% glucose, 2% agar, pH 6.5) and spores were attached thereon. The attached spores were suspended in an aqueous 0.9% NaCl solution containing 0.01 % Tween 80 to obtain a spore suspension. Ultraviolet ray was irradiated to the spore suspension to thereby induce a mutation, and the spore suspension was then spread on a minimum medium containing 5-fluoroorotic acid (5-FOA) (0.052% KCl, 0.152% $KH_2PO_4$, 0.6% $NaNO_3$, 0.1% trace element solution (0.004% $Na_2B_4O_7$•$10H_2O$, 0.04% $CuSO_4$•$5H_2O$, 0.08% $FePO_4$•$nH_2O$, 0.103% $MnSO_4$•$5H_2O$, 0.08% $Na_2MoO_4$•$2H_2O$, 0.8% $ZnSO_4$•$7H_2O$), 0.2% uridine, 1% glucose, 0.052% $MgSO_4$•$7H_2O$, 0.1% 5-FOA, 2% agar, pH 6.5) to obtain a 5-FOA resistant strain. Presence or absence of orotate phosphoribosyltransferase activity and orotidylate decarboxylase activity of the 5-FOA resistant strain were confirmed by a method of Belser, et al. (Methods in Enzymology Vol.51 P.135) and an orotidylate decarboxylase activity-defected mutant strain showing an uridine requiring property was obtained.

(5) Transformation to uridine requiring strain

The uridine requiring strain derived from Aspergillus oryzae BB-56 obtained above was cultured in an YPD medium containing uridine (1% yeast extract, 2% peptone, 2% glucose, 0.2% uridine, pH6.5) at 30°C for one night and the culture liquid was then passed through a glass filter to obtain a fungus body, and the fungus body was suspended in a protoplast preparation liquid (0.8 M NaCl, 10 mM sodium phosphate buffer, pH 6.0, 3 mg/ml Yatalase™, 0.3 mg/ml Novozymes 234 (Novo Nordisk Pharma Ltd.)) and treated at 30°C and 78 rpm for 1 hour and 30 minutes to perform a protoplastization treatment. The protoplast suspension was filtrated with a filter and the filtrate was centrifuged (780 g, 5 minute, room temperature) to obtain a precipitate. The precipitate was suspended in 10 mL of 0.8 M NaCl and the precipitate obtained by centrifugation (780 g, 5 minutes, room temperature) was suspended in 10 ml of 0.8 M NaCl. The number of protoplasts in the suspension was measured by microscopic observation and then centrifuged (780 g, 5 minutes, room temperature), and the precipitate was suspended in Sol I (0.8 M NaCl, 10 mM $CaCl_2$, 10 mM Tris-HCl, pH 8.0) to prepare about $2x10^8$/ml of a protoplast solution. The protoplast solution was added with 1/5 volume of a polyethylene glycol (PEG) solution (25% PEG 6000, 50 mM $CaCl_2$, 10 mM Tris-HCl, pH 8.0) to be mixed. 200 μl of the mixed solution was dividedly charged, thereto was added 13 μl of a solution (19 μg/μl) of 5 kbp of the gene fragment prepared above, which is obtained by linking a FAD-GDH gene and a pyrG gene, and the solution was suspended and then stood still in ice for 30 minutes. 1 mL of a PEG solution (25% PEG6000, 50 mM $CaCl_2$, 10 mM Tris-HCl, pH 8.0) was added to the suspension, and the mixture was suspended and stood still at room temperature for 15 minutes. Thereto was further added 8.5 mL of *Sol*I, and the mixture was suspended and centrifuged (780 g, 5 minutes, room temperature) to obtain a precipitate, and the precipitate was suspended in 1.2 mL of *Sol*I to obtain a protoplast suspension. 0.2 mL of the suspension was transferred to a petri dish, and thereafter a regenerating medium (0.052% KCl, 0.152% $KH_2PO_4$, 0.6% $NaNO_3$, 0.1 % trace element solution, 21.86% sorbitol, 1% glucose, 0.052% $MgSO_4$•$7H_2O$, 2% agar, pH 6.5) was poured to be solidified. After culture at 30°C for 5 days to 7 days, the grown transformant was isolated into a single fungus with a minimum medium (0.052% KCl, 0.152% $KH_2PO_4$, 0.6% $NaNO_3$, 0.1% trace element solution, 1% glucose, 0.052% $MgSO_4$•$7H_2O$, 2% agar, pH 6.5) to thus obtain a transformant of Aspergillus oryzae introduced with about 5 kbp of a ligated DNA fragment composed of an Aspergillus oryzae BB-56-derived FAD-GDH gene and pyrG.

Example 10

<Confirmation of productivity of FAD-GDH>

The transformant obtained in Example 9 was cultured and productivity of FAD-GDH was confirmed.

(1) Culture of transformant

A medium for preliminary culture constituted with the components below was dividedly charged in a conical flask and sterilized. Aspergillus oryzae BB-56 and the transformant obtained in Example 9 were respectively inoculated to the above described medium and preliminary culture was performed at 30°C and 200 rpm for 3 days.

(Medium components for preliminary culture)

Yeast extract (Becton, Dickinson Company) 0.2% (w/v)
Soybean peptone (DMV Co.) 1.0% (w/v)

Glucose (Wako Pure Chemical Industries, Ltd.) 2.0% (w/v)
$KH_2PO_4$ (Wako Pure Chemical Industries, Ltd.) 0.1 % (w/v)
$MgSO_4 \cdot 7H_2O$ (Sigma-Aldrich Japan K.K.) 0.05% (w/v)
pH 5.7

A medium for main culture constituted with the components below was dividedly charged in a conical flask and sterilized. After cooling, 1 mL of the culture liquid after preliminary culture described above was inoculated and main culture was performed at 30°C, 200 rpm for 4 days. After completion of the culture, the culture liquid was filtrated and a FAD-GDH activity of each filtrate was measured.

(Medium components for main culture)

Glucose 15.0%(w/v)
Meast P1G (ASAHI FOOD & HEALTHCARE CO., LTD.) 3.0% (w/v)
Soybean peptone 6.0% (w/v)
$KH_2PO_4$ 0.3% (w/v)
$K_2HPO_4$ (Wako Pure Chemical Industries, Ltd.) 0.2% (w/v)
4 mM Hydroquinone (Wako Pure Chemical Industries, Ltd.)
pH 6.0

(2) Measurement of FAD-GDH activity

FAD-GDH catalyzes a reaction of producing D-glucono-δ-lacton by oxidizing a hydroxyl group of glucose in the presence of an electron receptor. A FAD-GDH activity was measured in the following reaction system.
[Mathematical Formula 1]

(1) D-Glucose + PMS → D-glucono-δ-lacton + reduced PMS

(2) 2 Reduced PMS + NTB → 2PMS + Diformazan

PMS in the formula represents phenazine methanesulfate and NTB represents nitrotetrazorium blue.
In the reaction (1), reduced PMS was produced along with oxidization of glucose, and further in reaction (2), diformazan produced by reduction of NTB with reduced PMS was measured at a wavelength of 570 nm.
An enzyme activity was calculated with the calculating formula below.

[Mathematical Formula 2]

$$\text{Enzyme activity (u/mL)} = \frac{\Delta OD/\min(\Delta OD_{test} - \Delta OD_{blank}) \times Vt \times df}{20.1 \times 1.0 \times Vs}$$

Vt in the formula represents a total liquid amount, Vs represents a sample amount, 20.1 represents an absorbance coefficient per 0.5 μ mole of diformazan (cm2/0.5 μ mole), 1.0 represents a light path length (cm), and Df represents a diluting multiple, respectively.
2.55 mL of a 50 mM PIPES-NaOH buffer solution containing 0.22% (w/v) Triton X-100 at pH 6.5, 0.09 mL of a 1 M D-glucose solution, 0.2 mL of a 3 mM PMS solution, and 0.1 mL of a 6.6 mM NTB solution were mixed, the temperature was kept at 37°C for 5 minutes, and 0.1 mL of the above described culture filtrate was then added thereto to initiate a reaction. Diformazan having an absorption at 570 nm is produced along with progress of an enzyme reaction. An increase of an absorbance at 570 nm per 1 minute was measured and a FAD-GDH activity was measured (Fig. 3). As a result, the FAD-GDH activity of the Aspergillus oryzae BB-56-derived culture filtrate was 1.97 u/mL, and the FAD-GDH activity of the transformant-derived culture filtrate was 38.8 u/mL. Productivity of an enzyme was significantly improved to about 20 times due to transformation. The transformant was named Aspergillus oryzae 56-T. The transformant was deposited in an appointed deposit institution as described below and is easily available.

Deposit institution: Incorporated Administrative Agency, Patent Microorganisms Depositary, National Institute of Technology and Evaluation (2-5-8 Kazusakamatari, Kisarazu-shi, Chiba 292-0818, Japan)
Date of deposit: October 22, 2007
Accession number: NITE BP-438

Example 11

<Comparison of FAD-GDH production abilities of respective fungus strains belonging to Aspergillus oryzae>

FAD-GDH production abilities of 9 kinds of Aspergillus oryzae including Aspergillus oryzae BB-56, that is, Aspergillus oryzae BB-56, Aspergillus oryzae RIB40, Aspergillus oryzae IAM2603, Aspergillus oryzae IAM2609, Aspergillus oryzae IAM2628, Aspergillus oryzae IAM2683, Aspergillus oryzae IAM2736, Aspergillus oryzae IAM2706, and Aspergillus oryzae NBRC30113 were compared. Culture and measurement of FAD-GDH activities were conducted in accordance with the methods in Example 1 and Example 10. As a result, although culture liquids derived from Aspergillus oryzae RIB40 and Aspergillus oryzae IAM2609 were seemed to have FAD-GDH activities very weakly, the activities were a detection limit or less (Fig. 4).

Example 12

<Comparison between Aspergillus oryzae BB-56-derived FAD-GDH gene and other Aspergillus oryzae-derived FAD-GDH genes>

From comparison between the base sequences of Aspergillus oryzae BB-56-derived FAD-GDH found out in Example 8 and an Aspergillus oryzae RIB40-derived gene sequence (SEQ ID NO: 2) having a whole chain length of 3226 bp and encoding an amino acid sequence (SEQ ID NO: 1) of SEQ ID NO: 20494 in the sequence listing shown in JP 2005-176602 A, it was revealed that there is a nonhomologous region of 18 bases (6 amino acids) between the both sequences in regions that encode amino acids and do not contain intron (Figs. 5 and 6). Each chromosome DNA of 8 kinds of fungus strains belonging to Aspergillus oryzae, that is, Aspergillus oryzae RIB40, Aspergillus oryzae IAM2603, Aspergillus oryzae IAM2609, Aspergillus oryzae IAM2628, Aspergillus oryzae IAM2683, Aspergillus oryzae IAM2736, Aspergillus oryzae IAM2706, and Aspergillus oryzae NBRC30113 was prepared in accordance with the method of Example 5, these chromosome DNAs were used as templates, and PCR reactions were performed in accordance with the method of Example 7. These PCR reaction solutions were recovered in accordance with the method of Example 8, and the base sequences of the nonhomologous regions of 18 bases (6 amino acids) of an Aspergillus oryzae BB-56-derived FAD-GDH gene and an Aspergillus oryzae RIB40-derived FAD-GDH gene were determined using a synthetic primer FG-1 (SEQ ID NO: 5). These gene sequences were compared with the Aspergillus oryzae BB-56-derived FAD-GDH gene sequence (SEQ ID NO: 19) determined in Example 8. The result showed that only Aspergillus oryzae RIB40 and Aspergillus oryzae IAM2609 whose activities were not more than the detection limit in Example 11 had different 18 bases (6 amino acids) in a part of a base sequence of SEQ ID NO: 31 (corresponding amino acid sequence was represented by SEQ ID NO: 32) (Figs. 7 and 8).
The above result suggested that the revealed 18 bases (6 amino acid residues) give certain effects on productivity of FAD-GDH and stability of an enzyme.

Example 13

<Study of FAD-GDH production ability due to transformant of Aspergillus oryzae RIB40-derived FAD-GDH gene>

(1) Amplification of Aspergillus oryzae RIB40-derived FAD-GDH gene

Sequence information of an Aspergillus oryzae RIB40-derived FAD-GDH gene was obtained from gene information disclosed in JP 2005-176602 A to prepare synthetic primers, FG-E22 (SEQ ID NO: 23) and FG-R-Sal (SEQ ID NO: 24), which correspond to sequences of 5' and 3' ends. FG-E22 has a recognition site of a restricted enzyme, EcoT22I, and FG-R-Sal has a recognition site of a restricted enzyme, *Sal*I. A PCR reaction was performed using an Aspergillus oryzae RIB40-derived chromosome DNA prepared in Example 11 as a template. TAKARA LA Taq was used in the reaction. The PCR reaction was prepared so that each of primers of FG-E22 and FG-R-Sal has a concentration of 0.4 μM and a template genome has a concentration of 10 ng/μl. In the reaction, after reacting at 96°C for 2 minutes, reactions of (i) to (iii) constituted with (i) a reaction at 94°C for 30 seconds, (ii) a reaction at 53°C for 30 seconds, and (iii) a reaction at 72°C for 3 minutes and 30 seconds were repeated 35 times, and lastly, a reaction at 72°C for 10 minutes was performed. After completion of the reactions, the reaction solution was preserved at 4°C. The PCR amplified fragment was digested with restricted enzymes, EcoT22I and *Sal*I, and then supplied to agarose electrophoresis to separate the PCR amplified fragment and the primers, and the PCR amplified fragment, that is, an Aspergillus oryzae RIB40 strain-derived FAD-GDH gene fragment was extracted from an agarose gel using a GENECLEAN III Kit (Q-BIOgene Co.).

(2) Preparation of high expression promoter fragment

A PCR reaction was performed using pKF-PCSPb containing a high expression promoter gene fragment disclosed in JP 2003-319786 A as a template. TAKARA LA Taq was used in the reaction. Each synthesized primer of synthesized taaPf (SEQ ID NO: 25) and taaEcoT22R (SEQ ID NO: 26) was added so as to have a concentration of 1 μM and a template plasmid was added so as to have a concentration of 0.5 ng/μl to perform the PCR reaction. In the reaction cycle, after reacting at 94°C for 2 minutes, reactions constituted with (i) a reaction at 94°C for 30 seconds, (ii) a reaction at 60°C for 30 seconds, and (iii) a reaction at 72°C for 2 minutes were repeated 30 times, and lastly, a reaction at 72°C for 10 minutes was performed. After completion of the reactions, the sample was preserved at 4°C. The PCR amplified fragment was digested with restricted enzymes, *EcoR*I and *EcoT22*I, and then supplied to agarose electrophoresis to separate the PCR amplified fragment and the primers, and the PCR amplified fragment, that is, a high expression promoter gene fragment was extracted from an agarose gel using a GENECLEAN III Kit (Q-BIOgene Co.).

(3) Preparation of plasmid pCS-FG

The Aspergillus oryzae RIB40-derived FAD-GDH gene, the high expression promoter gene fragment, and plasmid pUC118 (TAKARA BIO INC.) treated with *EcoR*I and *Sal*I, which were prepared as described above, were ligated using a DNA Ligation Kit Ver. 2.1, and pCS-FG constructing plasmid pCS-FG was introduced in an E. coli DH5 strain competent cell to obtain a transformant of E. coli. The transformant was cultured to obtain plasmid pCS-FG using a GenElute™ Plasmid Miniprep Kit (Sigma-Aldrich Japan K.K.).

(4) Preparation of plasmid pCFPT

A PCR reaction was performed using pCS-FG prepared as described above as a template. KOD-plus was used in the reaction. Each of synthetic primers, FGH-F (SEQ ID NO: 27) and FGH-R (SEQ ID NO: 28) was added to have a concentration of 0.4 μM and a pCS-FG template plasmid was added to have a concentration of 0.5 ng/μl so that the PCR reaction solution was prepared. In a reaction cycle, after reacting at 96°C for 2 minutes, reactions (i) to (iii) constituted with (i) a reaction at 94°C for 30 seconds, (ii) a reaction at 54°C for 30 seconds, and (iii) a reaction at 68°C for 3 minutes and 30 seconds were repeated 30 times and, lastly, a reaction at 68°C for 10 minutes was performed. After completion of the reactions, the sample was preserved at 4°C. The PCR amplified fragment was digested with a restricted enzyme, *Hind*III, and then supplied to agarose electrophoresis to separate the PCR amplified fragment and the primers, and the amplified fragment was extracted from an agarose gel using a GENECLEAN III Kit (Q-BIOgene Co.). On the other hand, a vector pPTRI (TAKARA BIO INC.) was digested with *Hind*III and then 5'-dephosphorylated with an alkaline phosphatase (E. coli C75). The 5'-dephosphorylated vector and the above described PCR amplified fragment were ligated using a DNA Ligation Kit Ver. 2.1 to construct plasmid pCFPT. The pCFPT was introduced in an E. coli DH5 competent cell to obtain a transformant of E. coli. The transformant was cultured and plasmid pCFPT was extracted using a QIAfilter™ Plasmid Maxi Kit (QIAGEN K.K.).

(5) Transformation to Aspergillus oryzae RIB40 strain

Preparation of a protoplast solution of Aspergillus oryzae RIB40 was conducted in accordance with the method of Example 9. Introduction of genes into a protoplast was conducted in accordance with the method of Example 9 except that a gene to be introduced was 10 μl of a 1.8 μg/μl pCFPT solution. Regeneration of the protoplast after gene introduction was performed as follows. 0.2 mL of a protoplast suspension after gene introduction was transferred to a petri dish, and a regenerating medium (0.052% KCl, 0.152% $KH_2PO_4$, 0.6% $NaNO_3$, 0.1 % trace element solution, 21.86% sorbitol, 1% glucose, 0.052% $MgSO_4$•$7H_20$, 0.5 μg/ml pyrithiamine, 2% agar, pH 6.5) was poured to be solidified as a plate. After culture at 30°C for 5 days to 7 days, the grown pyrithiamine resistant strain was isolated into a single fungus with a minimum medium containing 0.5 μg/ml pyrithiamine (0.052% KCl, $KH_2PO_4$ 0.152%, 0.6% $NaNO_3$, 0.1% trace element solution, 1% glucose, 0.052% $MgSO_4$•$7H_2O$, 0.5 μg/ml pyrithiamine, 2% agar, pH 6.5) to thus obtain a transformant #75 having a plurality of Aspergillus oryzae RIB40-derived FAD-GDH genes on a chromosome.

Example 14

<Purification of Aspergillus oryzae RIB40-derived FAD-GDH>

The transformant #75 prepared in Example 13 was cultured in accordance with the method of Example 10 to obtain a culture liquid. Fungus bodies and other solid contents in the culture liquid were removed by diatomite filtration using 5.0 L of the culture liquid. 5.4 L of the purified liquid obtained through the operation was desalinated and concentrated with an ultrafilter membrane, and at the same time, buffer-exchanged to a 10 mM McIlvaine buffer solution at pH 5.0 to prepare 600 mL of a solution having a pH of 4.7 and a conductivity of 1.34 mS/cm. The concentrated liquid was applied to SP Sepharose™ Fast Flow equilibrated with a 10 mM McIlvaine buffer solution at pH 5.0 to adsorb FAD-GDH to a column. The column was washed with a 10 mM McIlvaine buffer solution at pH 5.0 and FAD-GDH was then eluted with a 10 mM McIlvaine buffer solution containing 0.1 M of NaCl at pH 5.0 to recover active fractions of FAD-GDH. The active fractions of FAD-GDH were combined to be one fraction, desalinated and concentrated with an ultrafilter membrane, and at the same time, buffer-exchanged to a 20 mM KH2PO4-NaOH buffer solution at pH 6.5 to prepare 300 mL of a concentrated liquid having a pH of 6.3 and a conductivity of 2.80 mS/cm. The concentrated liquid was applied to DEAE Sepharose™ CL-6B equilibrated with a 20mM KH2PO4-NaOH buffer solution at pH 6.5 and washed with a 20 mM $KH_2PO_4$-NaOH buffer solution at pH 6.5. The column pass-through and the washing liquid were all recovered and a column non-adsorbed fraction was recovered. This fraction was concentrated with an ultrafilter membrane to thus obtain a purified enzyme solution of Aspergillus oryzae RIB40-derived FAD-GDH. Productivity of an enzyme due to transformation was improved and purification from a large amount of the culture liquid was performed, thereby leading to success in narrowly obtaining 0.01 u/mL of a purified FAD-GDH solution.

Example 15

<Comparison of optical pH of FAD-GDH>

Optimal pHs of Aspergillus oryzae BB-56-derived FAD-GDH prepared in Example 2 and Aspergillus oryzae RIB40-derived FAD-GDH prepared in Example 14 were compared.
2.55 mL of a 100 mM McIlvaine buffer solution containing 0.22% (w/v) Triton X-100 (pH was adjusted to 4.5, 5.0, 6.0, 7.0, or 8.0), 0.1 mL of a 1 M D-glucose solution, 0.2 mL of a 3 mM PMS solution, and 0.1 mL of a 6.6 mM NTB solution were mixed and the temperature was kept at 37°C for 5 minutes, and then, each 150 μl of the mixed solution was dividedly charged in a 96-well microplate and 5 μl of an Aspergillus oryzae BB-56-derived or Aspergillus oryzae RIB40-derived purified FAD-GDH solution was added to initiate a reaction. Diformazan produced by an enzyme reaction was measured at an absorbance of 570 nm and a yield of diformazan per 1 minute was measured to thereby measure an enzyme activity. POWERSCAN HT (Dainippon Pharmaceutical Co., Ltd.) was used in the measurement. An optimal pH of Aspergillus oryzae BB56-derived purified FAD-GDH was around 7 (Fig. 9A), and an optimal pH of Aspergillus oryzae RIB40-derived purified FAD-GDH was 6.0 (Fig. 9B). The result showed that the optimal pHs of the both enzymes were different.

Example 16

<Comparison of pH stability of FAD-GDH>

Aspergillus oryzae BB-56-derived or Aspergillus oryzae RIB40-derived purified FAD-GDH solutions was each diluted with a 0.1 M acetate buffer solution at pH 3, the same buffer solution at pH 4, a 0.1 M McIlvaine buffer solution at pH 4.5, the same buffer solution at pH 5.0, the same buffer solution at pH 6.0, the same buffer solution at pH 7.0, the same buffer solution at pH 8.0, or a 0.1 M $Na_2CO_3$-$NaHCO_3$ buffer solution at pH 9.5, and treated at 37°C for 30 minutes at each pH. An each treated liquid was diluted with a 0.2 M $KH_2PO_4$-NaOH buffer solution at pH 6.5, and FAD-GDH activities were measured in the same method as Example 15. As a control, an activity of purified FAD-GDH that was diluted with a 0.1 M $KH_2PO_4$-NaOH buffer solution at pH 6.5 and cooled with ice to be preserved was also measured and, when the activity was assumed to be 100%, a relative activity was calculated. As a result, Aspergillus oryzae BB-56-derived purified FAD-GDH kept 80% or more of an activity within the pH range from 3.0 to 7.0 (Fig. 10A). On the other hand, it was shown that, for pH stability, Aspergillus oryzae RIB40-derived purified FAD-GDH was stable only within the pH range from 4.0 to 5.0 and the stable pH range is narrow (Fig. 10B).

Example 17

<Comparison of temperature stability of FAD-GDH>

Aspergillus oryzae BB-56-derived or Aspergillus oryzae RIB40-derived purified FAD-GDH was diluted with a 50 mM PIPES-NaOH buffer solution containing 0.22% of (w/v) Triton X-100 at pH 6.5, and treated at respective specified temperatures, that is, 4°C, 30°C, 40°C, 50°C, and 60°C, for 20 minutes. After cooling in ice, a FAD-GDH activity of an each treated liquid was measured in the same method as that in Example 15. As a control, a FAD-GDH activity of an enzyme without a thermal treatment was also measured and, when the activity was assumed to be 100%, a relative activity was calculated. As a result, the Aspergillus oryzae BB-56-derived purified FAD-GDH kept approximately 100% of an activity until 40°C (Fig. 11A). On the other hand, the activity of the Aspergillus oryzae RIB40-derived purified FAD-GDH was drastically dropped at 30°C or more (Fig. 11B).

Example 18

<Comparison of substrate specificity of FAD-GDH>

2.55 mL of a 50 mM PIPES-NaOH buffer solution (pH 6.5) containing 0.22% (w/v) Triton X-100, 0.09 mL of a solution of a 1 M substrate (D-glucose, maltose, galactose, xylose, maltotriose, maltohexaose, or maltopentaose), 0.2 mL of a 3 mM PMS solution, and 0.1 mL of a 6.6 mM NTB solution were mixed and the temperature was kept at 37°C for 5 minutes, and then, each 150 μl of the mixed solution was dividedly charged in a 96-well microplate, and 5 μl (activity) of an Aspergillus oryzae BB-56-derived or Aspergillus oryzae RIB40-derived purified FAD-GDH solution was added to initiate a reaction. Diformazan produced by an enzyme reaction was measured at an absorbance of 570 nm and a yield of diformazan per 1 minute was measured to thereby measure an enzyme activity. POWERSCAN HT (Dainippon Pharmaceutical Co., Ltd.) was used in the measurement. A relative activity for each substrate was calculated when a reaction rate for D-glucose was assumed to be 100%. The result showed that Aspergillus oryzae RIB40-derived purified FAD-GDH was acted on not only glucose but also maltose, galactose, and maltohexaose, and thus, substrate specificity thereof was lower than that of the Aspergillus oryzae BB-56-derived FAD-GDH (Fig. 12).

As described above, the results of Examples 15 to 18 revealed that 18 base sequences (6 amino acids) that do not correspond between an Aspergillus oryzae BB-56-derived FAD-GDH gene sequence and an Aspergillus oryzae RIB40-derived FAD-GDH gene sequence give effects on pH stability, thermal stability, and substrate specificity of an enzyme.

Example 19

<Preparation of transformant of yeast introduced with cDNA of FAD-GDH>

(1) Preparation of Aspergillus oryzae BB-56 or Aspergillus oryzae RIB40-derived cDNA

50 mL of a medium constituted with the components below was dividedly charged in a 300 mL-volume conical flask and sterilized at 121°C for 20 minutes. After cooling, each of Aspergillus oryzae BB-56 and Aspergillus oryzae RIB40 was inoculated and cultured at 30°C and 200 rpm for 3 days.

(Medium components)

Glucose 15.0% (w/v)
Meast P1G (ASAHI FOOD & HEALTHCARE CO., LTD.) 3.0% (w/v)
Soybean peptone 6.0% (w/v)
$KH_2PO_4$ 0.3% (w/v)
$K_2HPO_4$ (Wako Pure Chemical Industries, Ltd.) 0.2% (w/v)
4 mM Hydroquinone (Wako Pure Chemical Industries, Ltd.)
pH 6.0

Fungus bodies recovered through filtration of a culture liquid after culture was rapidly frozen with liquid nitrogen and crushed. The crushed fungus bodies were added with 2 ml of a TRIzol reagent (Invitrogen Corp.) and further crushed. The crushed fungus bodies were adjusted back to room temperature, the suspension of the crushed fungus bodies was collected in a 1.5 ml-volume tube, and whole RNA was obtained in accordance with a method described in an attached instruction manual. Then, a Micro FastTrack 2.0 Kit (Invitrogen Corp.) was used and mRNA was purified in accordance with a method described in an attached instruction manual. A Takara RNA LA PCR™ kit (AMV) ver. 1.1 was used and the mRNA was reverse-transcribed to thus obtain a cDNA solution.

(2) Preparation of cDNA of FAD-GDH

Two kinds of cDNAs prepared as described above were used as templates, and cDNAs of FAD-GDH were amplified by a PCR reaction. KOD-Plus was used in the reaction, and components of the reaction solution were in accordance with an attached instruction manual. 1 μM of each of primers of FADGDH-1 (SEQ ID NO: 29) and FADGDH-2 (SEQ ID NO: 30), and 1 μl of the cDNA solution as a template cDNA were added to the reaction system and a PCR reaction was performed. In the reaction cycle, after reacting at 94°C for 2 minutes, reactions of (i) to (iii) constituted with (i) a reaction at 94°C for 30 seconds, (ii) a reaction at 60°C for 30seconds, and (iii) a reaction at 68°C for 2 minutes were repeated 35 times. After completion of the reactions, the reaction solution was preserved at 4°C.

(3) Preparation of yeast transformant

The two kinds of cDNA fragments of FAD-GDH obtained as described above were respectively digested with restricted enzymes, *Hind*III and *Bam*HI. The reaction solutions after the reaction with the restricted enzymes were supplied to agarose electrophoresis to separate a desired fragment and extract from an agarose gel using a GENECLEAN III Kit (Q-BIOgene Co.). These DNA fragments were each ligated with a yeast expression vector, pYES2 (Invitrogen Corp.) that was treated with restricted enzymes, *Hind*III and *Bam*HI, in advance to thus prepare pYESGDH having a cDNA of Aspergillus oryzae BB-56-derived FAD-GDH and pYES2-RFGC having a cDNA of Aspergillus oryzae RIB40-derived FAD-GDH. The ligation was performed using a DNA Ligation Kit Ver. 2.1. pYESGDH and pYES2-RFGC were respectively introduced in a Saccharomyces cerevisiae INVSc strain (Invitrogen Corp.) to obtain transformants. The transformation was performed in a lithium acetate method described in an attached instruction manual.

(4) Confirmation of base sequence of genetically transduced cDNA fragment

It was confirmed that the desired cDNA fragments were correctly introduced in the above described two kinds of transformants. The cDNA fragments of two types of FAD-GDH digested with restricted enzymes, *Hind*III and *BamH*I, which were obtained in the above described (3), were ligated with a vector pBR322 (Toyobo Co., Ltd.) treated with restricted enzymes, *Hind*III and *Bam*HI to thus construct (i) pGDH having a cDNA of Aspergillus oryzae BB-56-derived FAD-GDH and (ii) pRFGC having a cDNA of Aspergillus oryzae RIB40-derived FAD-GDH. The linking was performed using a DNA Ligation Kit Ver.2.1. Each of pGDH and pRFGC was introduced in an E. coli DH5 strain competent cell to thus obtain a transformant of E. coli. These two kinds of the transformants were respectively cultured to thus obtain plasmids, pGDH and pRFGC, using a GenElute™ Plasmid Miniprep Kit (Sigma-Aldrich Japan K.K.). The base sequences of pGDH and pRFGC were determined in accordance with the method of Example 8. As a result, it could be confirmed that pGDH was a sequence in which a promoter sequence, a terminator sequence and an intron sequence of SEQ ID NO: 19 were dropped (cDNA sequence in the upper column in Fig. 5) and has a cDNA fragment (SEQ ID NO: 33) in which the 4th base of C was replaced with G. Substitution of the base was generated due to introduction of a restricted enzyme NcoI recognition site. An amino acid sequence (SEQ ID NO: 34) corresponding to SEQ ID NO: 33 was only different from SEQ ID NO: 20 with respect that the 2nd amino acid was replaced to Val. On the other hand, it could be confirmed that pRFGC was a sequence in which a promoter sequence, a terminator sequence and an intron sequence of SEQ ID NO: 2 were dropped (cDNA sequence in the lower column in Fig. 5) and has a cDNA fragment in which the 4th base of C was replaced with G. Substitution of the base was generated due to introduction of a restricted enzyme NcoI recognition sequence. From the above described results, it was confirmed that (i) a cDNA of Aspergillus oryzae BB-56-derived FAD-GDH and (ii) a cDNA of Aspergillus oryzae RIB40-derived FAD-GDH, respectively has been correctly introduced into the transformants of the yeasts obtained in the above described (4).

Example 20

<Comparison of production of FAD-GDH due to transformant of yeast and enzyme production ability>

Two kinds of the transformants obtained in Example 19 were cultured to produce FAD-GDH. Culture was performed in an induction culture method with Galactose, which is recommended in an attached instruction manual. In order to verify production of FAD-GDH outside and inside fungus bodies after the culture, FAD-GDH activities of the culture supernatant and the fungus body crushed liquid were measured. Measurement of the FAD-GDH activities was performed as follows.

(Measurement of FAD-GDH activity)

2.55 mL of a 50 mM PIPES-NaOH buffer solution (pH 6.5) containing 0.22% (w/v) Triton X-100, 0.09 mL of a 1 M D-glucose solution, 0.2 mL of a 3 mM PMS solution, and 0.1 mL of a 6.6 mM NTB solution were mixed and the temperature was kept at 37°C for 5 minutes, then, 150 μl of this mixed solution was dividedly charged in a 96-well microplate, and thereto was added 5 μl of the culture supernatant or the fungus body crushed liquid, that is, a culture supernatant or a fungus body crushed liquid of a yeast transformed with a cDNA of Aspergillus oryzae BB-56-derived FAD-GDH or a yeast transformed with a cDNA of Aspergillus oryzae RIB40-derived FAD-GDH to initiate a reaction. Diformazan produced by an enzyme reaction was measured at an absorbance of 570 nm and a yield of diformazan per 1 minute was measured to thus measure a FAD-GDH activity. POWERSCAN HT (Dainippon Pharmaceutical Co., Ltd.) was used in the measurement.

As a result, a FAD-GDH activity was detected in both outside and inside of fungus bodies in the yeast transformed with an Aspergillus oryzae BB-56-derived cDNA. On the other hand, a FAD-GDH activity was not detected in either outside or inside of fungus bodies in the yeast transformed with an Aspergillus oryzae RIB40-derived cDNA (Fig. 13).

Example 21

<Purification of FAD-GDH>

Purification of FAD-GDH was tried from two kinds of the culture supernatants obtained in Example 20. Each of the culture supernatants was supplied to dialysis using a 10 mM acetate Na buffer solution at pH 5.0 as an external dialysate. The solution after dialysis was applied to HiTrap™ SP Sepharose Fast Flow (GE Healthcare Bio-Sciences Co., Ltd.) equilibrated with a 10 mM acetate Na buffer solution at pH 5.0 to adsorb FAD-GDH to a column. The column was washed with a 10 mM acetate Na buffer solution at pH 5.0, and FAD-GDH was then eluted with a 10 mM acetate Na buffer solution containing 0.1 M of NaCl at pH 5.0 to recover a fraction showing a FAD-GDH activity. Measurement of FAD-GDH activities was conducted in accordance with the method of Example 20.
The result showed that most of Aspergillus oryzae BB-56 derived FAD-GDH was detected from a column adsorbed eluted fraction (Fig. 14). On the other hand, it was shown that an activity of Aspergillus oryzae RIB40-derived FAD-GDH was not detected from either a column non-adsorbed fraction or a column adsorbed fraction (Fig. 15).

Example 22

<Removal of sugar chain of FAD-GDH>

Among the purified enzyme fractions obtained by the purification operation in Example 21, a fraction NO. 18 having the highest relative activity of an Aspergillus oryzae BB-56-derived FAD-GDH activity (Fig. 14), a purified fraction NO. 18 of Aspergillus oryzae RIB40-derived FAD-GDH (Fig. 15), and FAD-GDH purified from a culture supernatant derived from wild type Aspergillus oryzae BB-56 prepared in Example 2 were used as samples of sugar chain removal. Each sample was boiled in a 50 mM acetate buffer solution at pH 5.5 containing 0.1 M β-mercaptoethanol and 0.02% SDS for 5 minutes and thereto was added 0.05 U of endoglycosidase H (Roche Diagnostics K.K.) to react at 37°C for one night. After the reaction, the reaction solution was applied to SDS-PAGE using PhastSystem (GE Healthcare Bio-Sciences Co., Ltd.) and the gel was silver-stained in accordance with an attached method in an instruction manual. The result showed that a yeast in which a cDNA encoding Aspergillus oryzae BB-56-derived FAD-GDH has been introduced produced the same protein as the wild-type Aspergillus oryzae BB-56-derived FAD-GDH (lanes 1 to 4 in Fig. 16). On the other hand, it was shown that a yeast in which a cDNA encoding Aspergillus oryzae RIB40-derived FAD-GDH has been introduced did not express FAD-GDH (lanes 5 and 6 in Fig. 16).

As described above, the results of respective examples revealed that 18 nucleic acid bases (6 amino acids) that do not correspond between a gene encoding Aspergillus oryzae BB-56-derived FAD-GDH and a gene encoding Aspergillus oryzae RIB40-derived FAD-GDH give effects on an optical pH, pH stability, thermal stability and substrate specificity of FAD-GDH, and production ability of an enzyme.

Industrial Applicability

According to the transformant of the present invention, FAD-GDH excellent in substrate specificity can be produced at high efficiency and in a large amount. FAD-GDH produced by the transformant of the present invention makes it possible to measure a glucose level more accurately and is suitable for a measurement of a blood sugar level and a measurement of a glucose concentration in foods (such as seasonings and beverages).

## Claims

1. A transformant, wherein the transformant is obtained by transforming *Aspergillus oryzae* and wherein the *Aspergillus oryzae* is a uridine requiring strain, in which a DNA encoding a flavin adenine dinucleotide-binding glucose dehydrogenase selected from the group consisting of the following (a) to (c) has been introduced:

   (a) a DNA encoding the amino acid sequence of SEQ ID NO: 20;
   (b) a DNA consisting of the base sequence of SEQ ID NO: 19;
   (c) a DNA having a base sequence homologous to the base sequence of SEQ ID NO: 19 and encoding a protein having a flavin adenine dinucleotide-binding glucose dehydrogenase activity.

2. The transformant according to claim 1 as deposited under the accession number NITE BP-43 8.

3. A method for producing a flavin adenine dinucleotide-binding glucose dehydrogenase, comprising the following steps (1) and (2):

   (1) a step of culturing the transformant according to claim 1 or 2 under the condition of producing a protein encoded by the DNA; and
   (2) a step of recovering the produced protein.

## Patentansprüche

1. Transformant, wobei der Transformant durch Transformieren des *Aspergillus oryzae* erhalten wird, und wobei *Aspergillus oryzae* ein Uridin benötigter Stamm ist, in den eine Flavin-Adenin-Dinukleotid bindende Glucose-Dehydrogenase kodierende DNA eingeführt wurde, ausgewählt aus der Gruppe, bestehend aus den folgenden (a) bis (c):

   (a) eine DNA, die die Aminosäuresequenz von SEQ ID NO: 20 kodiert;
   (b) eine DNA, bestehend aus der Basensequenz von SEQ ID NO: 19;
   (c) eine DNA, die eine Basensequenz homolog zu der Basensequenz von SEQ ID NO: 19 aufweist und ein Protein mit einer Flavin-Adenin-Dinukleotid bindenden Glucose-Dehydrogenase-Aktivität kodiert.

2. Transformant nach Anspruch 1, wie unter der Zugangsnummer NITE BP-438 hinterlegt.

3. Verfahren zur Herstellung einer Flavin-Adenin-Dinukleotid bindenden Glucose-Dehydrogenase, umfassend die folgenden Schritte (1) und (2):

(1) einen Schritt der Kultivierung des Transformants nach Anspruch 1 oder 2 unter der Bedingung der Herstellung eines durch die DNA kodierten Proteins; und
(2) einen Schritt der Gewinnung des hergestellten Proteins.

## Revendications

1. Transformant, où le transformant est obtenu par transformation *d'Aspergillus oryzae* et où *l'Aspergillus oryzae* est une souche nécessitant de l'uridine, dans laquelle un ADN codant pour un glucose déshydrogénase liant le flavine-adénine-dinucléotide choisi dans le groupe constitué des (a) à (c) suivants a été introduit:

(a) un ADN codant pour la séquence d'acides aminés de SEQ ID NO: 20;
(b) un ADN constitué de la séquence de bases de SEQ ID NO: 19;
(c) un ADN ayant une séquence de bases homologue à la séquence de bases de SEQ ID NO: 19 et codant pour une protéine possédant une activité glucose déshydrogénase liant le flavine-adénine-dinucléotide.

2. Transformant selon la revendication 1 tel que déposé sur le numéro d'accession NITE BP-438.

3. Procédé de production d'un glucose déshydrogénase liant le flavine-adénine-dinucléotide, comprenant les étapes (1) et (2) suivantes:

(1) une étape de culture du transformant selon la revendication 1 ou 2 sous la condition de produire une protéine codée par l'ADN; et
(2) une étape de récupération de la protéine produite.

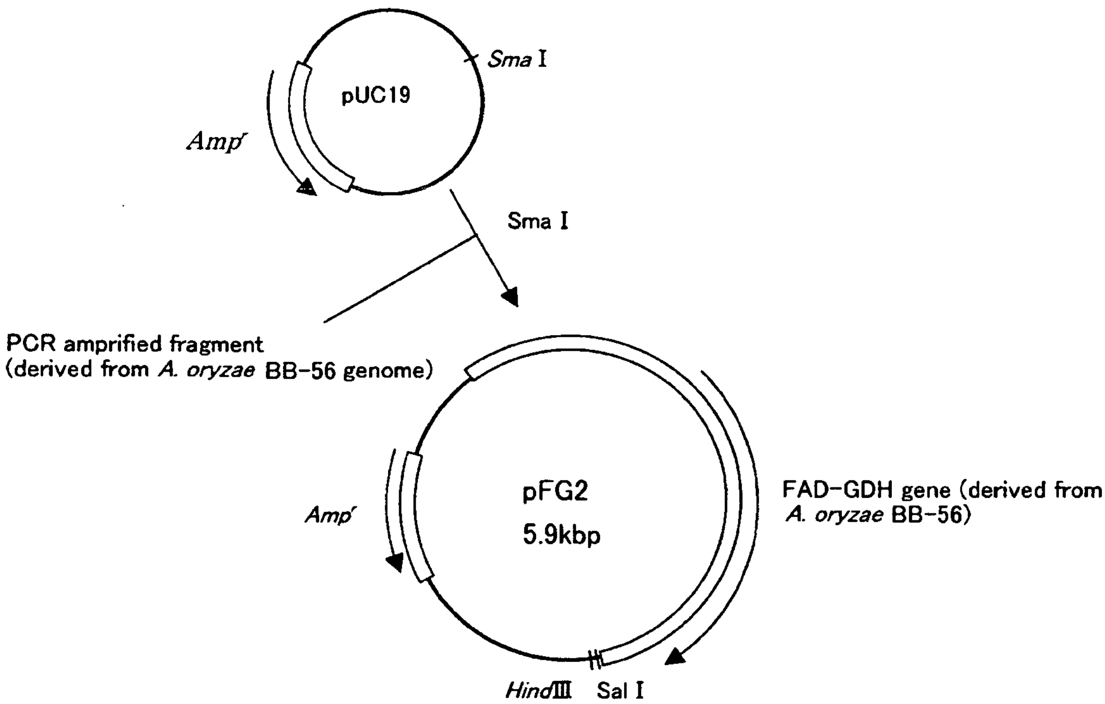

pUC19
*Sma* I
*Amp'*
Sma I
PCR amprified fragment
(derived from *A. oryzae* BB-56 genome)
pFG2
5.9kbp
*Amp'*
FAD-GDH gene (derived from
*A. oryzae* BB-56)
*Hind*III Sal I


*Fig. 1*

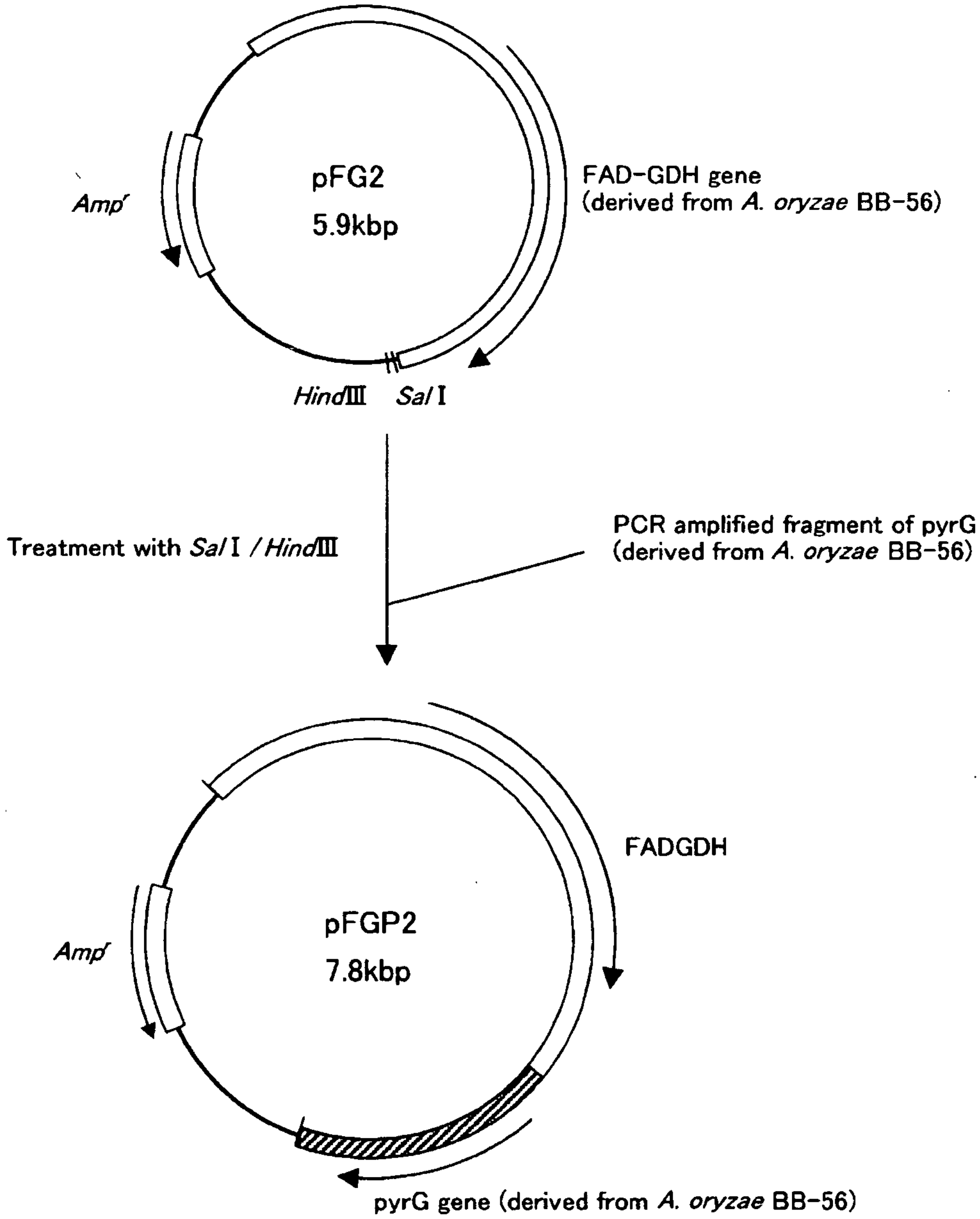
pFG2
5.9kbp
Amp^r
FAD-GDH gene
(derived from A. oryzae BB-56)
HindIII
SalI
Treatment with SalI / HindIII
PCR amplified fragment of pyrG
(derived from A. oryzae BB-56)
pFGP2
7.8kbp
Amp^r
FADGDH
pyrG gene (derived from A. oryzae BB-56)

*Fig. 2*

| Name of fungus | Activity (u/ml) |
|---|---|
| Aspergillus oryzae BB−56 | 1.97 |
| Aspergillus oryzae 56−T | 38.8 |

*Fig. 3*

| Name of fungus | Activity (u/ml) |
|---|---|
| Aspergillus oryzae BB-56 | 1.97 |
| Aspergillus oryzae RIB40 | ND |
| Aspergillus oryzae IAM2603 | 0.11 |
| Aspergillus oryzae IAM2609 | ND |
| Aspergillus oryzae IAM2628 | 0.87 |
| Aspergillus oryzae IAM2683 | 0.41 |
| Aspergillus oryzae IAM2736 | 0.20 |
| Aspergillus oryzae IAM2706 | 0.75 |
| Aspergillus oryzae NBRC30113 | 2.12 |

ND; No Detectable

*Fig. 4*

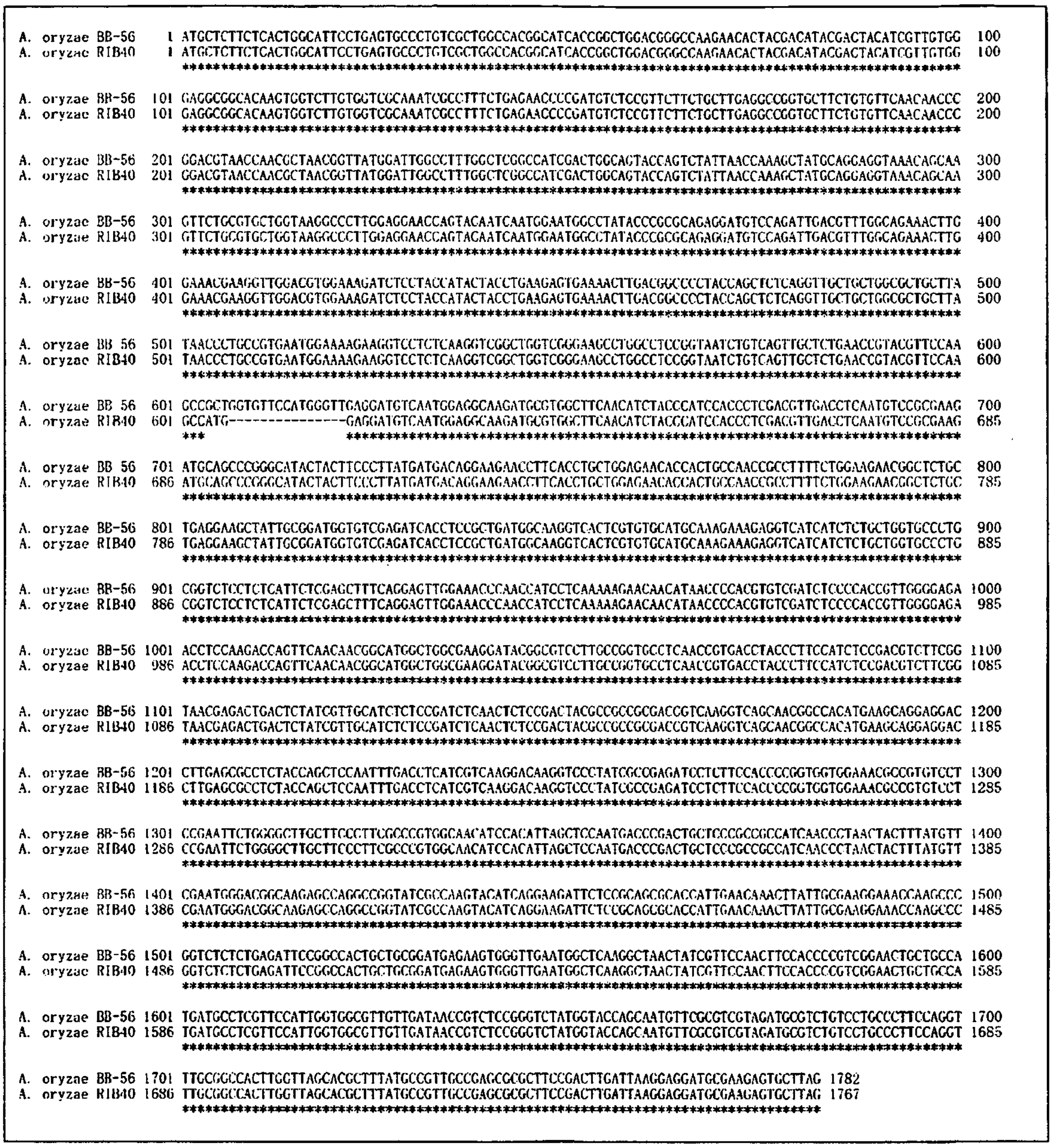

*Fig. 5*

```
A. oryzae BB-56    1 MLFSLAFLSALSLATASPAGRAKNTTTYDYIVVGGGTSGLVVANRLSENPDVSVLLLEAG  60
A. oryzae RIB40    1 MLFSLAFLSALSLATASPAGRAKNTTTYDYIVVGGGTSGLVVANRLSENPDVSVLLLEAG  60
                     ************************************************************

A. oryzae BB-56   61 ASVFNNPDVTNANGYGLAFGSAIDWQYQSINQSYAGGKQQVLRAGKALGGTSTINGMAYT 120
A. oryzae RIB40   61 ASVFNNPDVTNANGYGLAFGSAIDWQYQSINQSYAGGKQQVLRAGKALGGTSTINGMAYT 120
                     ************************************************************

A. oryzae BB-56  121 RAEDVQIDVWQKLGNEGWTWKDLLPYYLKSENLTAPTSSQVAAGAAYNPAVNGKEGPLKV 180
A. oryzae RIB40  121 RAEDVQIDVWQKLGNEGWTWKDLLPYYLKSENLTAPTSSQVAAGAAYNPAVNGKEGPLKV 180
                     ************************************************************

A. oryzae BB-56  181 GWSGSLASGNLSVALNRTFQAAGVPWVEDVNGGKMRGFNIYPSTLDVDLNVREDAARAYY 240
A. oryzae RIB40  181 GWSGSLASGNLSVALNRTFQAM-----EDVNGGKMRGFNIYPSTLDVDLNVREDAARAYY 235
                     *********************      *********************************

A. oryzae BB-56  241 FPYDDRKNLHLLENTTANRLFWKNGSAEEAIADGVEITSADGKVTRVHAKKEVIISAGAL 300
A. oryzae RIB40  236 FPYDDRKNLHLLENTTANRLFWKNGSAEEAIADGVEITSADGKVTRVHAKKEVIISAGAL 295
                     ************************************************************

A. oryzae BB-56  301 RSPLILELSGVGNPTILKKNNITPRVDLPTVGENLQDQFNNGMAGEGYGVLAGASTVTYP 360
A. oryzae RIB40  296 RSPLILELSGVGNPTILKKNNITPRVDLPTVGENLQDQFNNGMAGEGYGVLAGASTVTYP 355
                     ************************************************************

A. oryzae BB-56  361 SISDVFGNETDSIVASLRSQLSDYAAATVKVSNGHMKQEDLERLYQLQFDLIVKDKVPIA 420
A. oryzae RIB40  356 SISDVFGNETDSIVASLRSQLSDYAAATVKVSNGHMKQEDLERLYQLQFDLIVKDKVPIA 415
                     ************************************************************

A. oryzae BB-56  421 EILFHPGGGNAVSSEFWGLLPFARGNIHISSNDPTAPAAINPNYFMFEWDGKSQAGIAKY 480
A. oryzae RIB40  416 EILFHPGGGNAVSSEFWGLLPFARGNIHISSNDPTAPAAINPNYFMFEWDGKSQAGIAKY 475
                     ************************************************************

A. oryzae BB-56  481 IRKILRSAPLNKLIAKETKPGLSEIPATAADEKWVEWLKANYRSNFHPVGTAAMMPRSIG 540
A. oryzae RIB40  476 IRKILRSAPLNKLIAKETKPGLSEIPATAADEKWVEWLKANYRSNFHPVGTAAMMPRSIG 535
                     ************************************************************

A. oryzae BB-56  541 GVVDNRLRVYGTSNVRVVDASVLPFQVCGHLVSTLYAVAERASDLIKEDAKSA 593
A. oryzae RIB40  536 GVVDNRLRVYGTSNVRVVDASVLPFQVCGHLVSTLYAVAERASDLIKEDAKSA 588
                     *****************************************************
```

*Fig. 6*

```
A. oryzae BB-56        .....TGGTCGGGAAGCCTGGCCTCCGGTAATCTGTCAGTTGCTCTGAACCGTACGTTC
A. oryzae RIB40        .....TGGTCGGGAAGCCTGGCCTCCGGTAATCTGTCAGTTGCTCTGAACCGTACGTTC
A. oryzae IAM2603      .....TGGTCGGGAAGCCTGGCCTCCGGTAATCTGTCAGTTGCTCTGAACCGTACGTTC
A. oryzae IAM2609      .....TGGTCGGGAAGCCTGGCCTCCGGTAATCTGTCAGTTGCTCTGAACCGTACGTTC
A. oryzae IAM2628      .....TGGTCGGGAAGCCTGGCCTCCGGTAATCTGTCAGTTGCTCTGAACCGTACGTTC
A. oryzae IAM2683      .....TGGTCGGGAAGCCTGGCCTCCGGTAATCTGTCAGTTGCTCTGAACCGTACGTTC
A. oryzae IAM2636      .....TGGTCGGGAAGCCTGGCCTCCGGTAATCTGTCAGTTGCTCTGAACCGTACGTTC
A. oryzae IAM2706      .....TGGTCGGGAAGCCTGGCCTCCGGTAATCTGTCAGTTGCTCTGAACCGTACGTTC
A. oryzae NBRC30113    .....TGGTCGGGAAGCCTGGCCTCCGGTAATCTGTCAGTTGCTCTGAACCGTACGTTC
                            ******************************************************

A. oryzae BB-56        CAAGCCGCTGGTGTTCCATGGGTTGAGGATGTCAATGGAGGCAAGATGCGTGGC.....
A. oryzae RIB40        CAAGCCATG---------------GAGGATGTCAATGGAGGCAAGATGCGTGGC.....
A. oryzae IAM2603      CAAGCCGCTGGTGTTCCATGGGTTGAGGATGTCAATGGAGGCAAGATGCGTGGC.....
A. oryzae IAM2609      CAAGCCATG---------------GAGGATGTCAATGGAGGCAAGATGCGTGGC.....
A. oryzae IAM2628      CAAGCCGCTGGTGTTCCATGGGTTGAGGATGTCAATGGAGGCAAGATGCGTGGC.....
A. oryzae IAM2683      CAAGCCGCTGGTGTTCCATGGGTTGAGGATGTCAATGGAGGCAAGATGCGTGGC.....
A. oryzae IAM2636      CAAGCCGCTGGTGTTCCATGGGTTGAGGATGTCAATGGAGGCAAGATGCGTGGC.....
A. oryzae IAM2706      CAAGCCGCTGGTGTTCCATGGGTTGAGGATGTCAATGGAGGCAAGATGCGTGGC.....
A. oryzae NBRC30113    CAAGCCGCTGGTGTTCCATGGGTTGAGGATGTCAATGGAGGCAAGATGCGTGGC.....
                       ******                  ******************************
```

*Fig. 7*

```
A. oryzae BB-56       .....WSGSLASGNLSVALNRTFQAAGVPWVEDVNGGKMRG.....
A. oryzae RIB40       .....WSGSLASGNLSVALNRTFQAM-----EDVNGGKMRG.....
A. oryzae IAM2603     .....WSGSLASGNLSVALNRTFQAAGVPWVEDVNGGKMRG.....
A. oryzae IAM2609     .....WSGSLASGNLSVALNRTFQAM-----EDVNGGKMRG.....
A. oryzae IAM2628     .....WSGSLASGNLSVALNRTFQAAGVPWVEDVNGGKMRG.....
A. oryzae IAM2683     .....WSGSLASGNLSVALNRTFQAAGVPWVEDVNGGKMRG.....
A. oryzae IAM2736     .....WSGSLASGNLSVALNRTFQAAGVPWVEDVNGGKMRG.....
A. oryzae IAM2706     .....WSGSLASGNLSVALNRTFQAAGVPWVEDVNGGKMRG.....
A. oryzae NBRC30113   .....WSGSLASGNLSVALNRTFQAAGVPWVEDVNGGKMRG.....
                           ********************      **********
```

*Fig. 8*

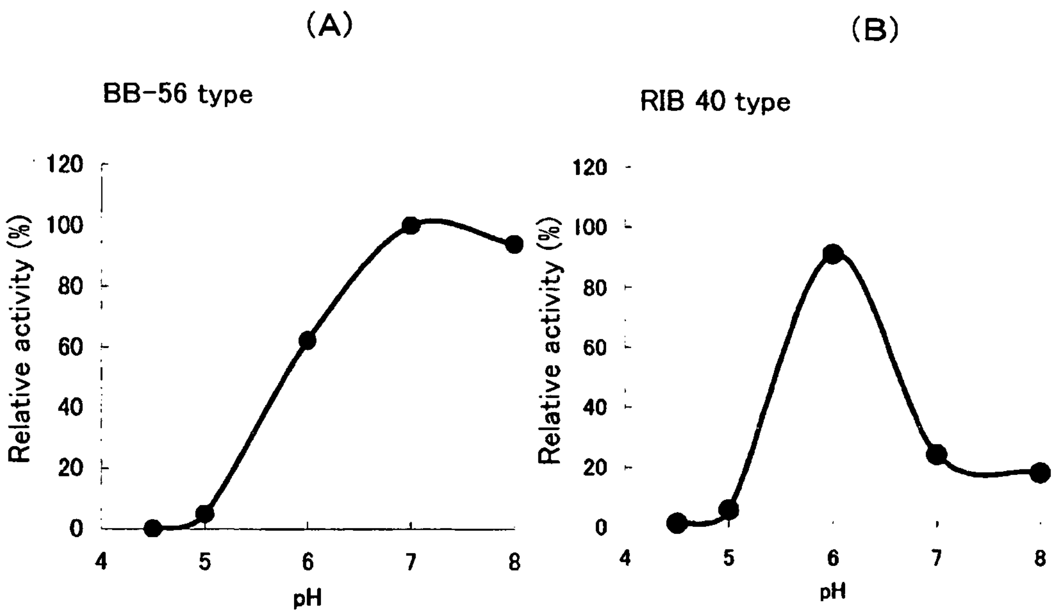
(A)
BB-56 type
Relative activity (%)
120
100
80
60
40
20
0
4
5
6
7
8
pH
(B)
RIB 40 type
Relative activity (%)
120
100
80
60
40
20
0
4
5
6
7
8
pH

*Fig. 9*

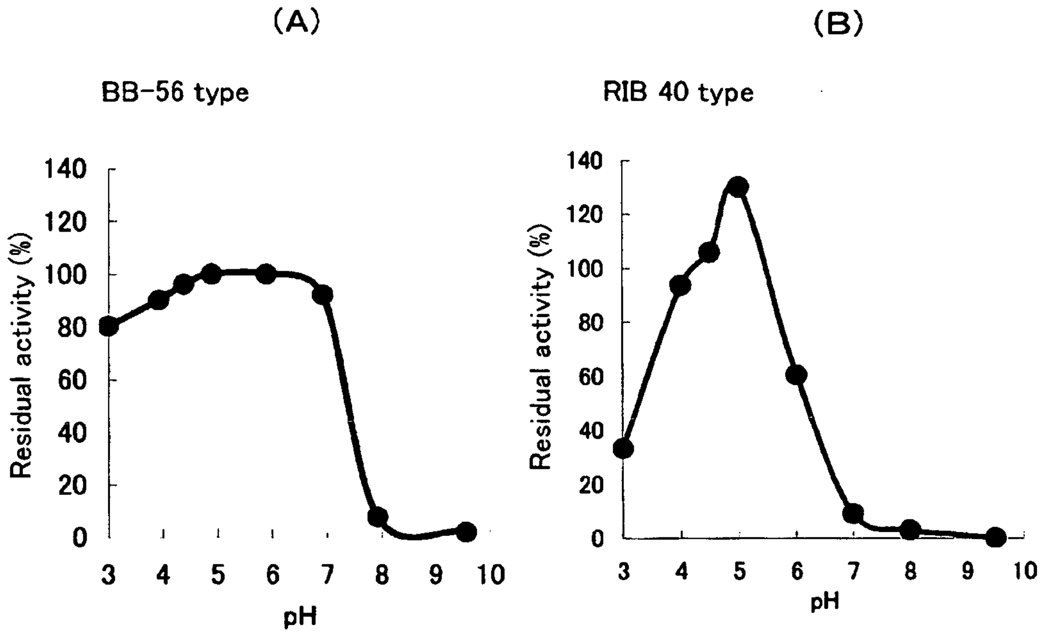
(A)
BB-56 type
Residual activity (%)
140
120
100
80
60
40
20
0
3 4 5 6 7 8 9 10
pH
(B)
RIB 40 type
Residual activity (%)
140
120
100
80
60
40
20
0
3 4 5 6 7 8 9 10
pH

*Fig. 10*

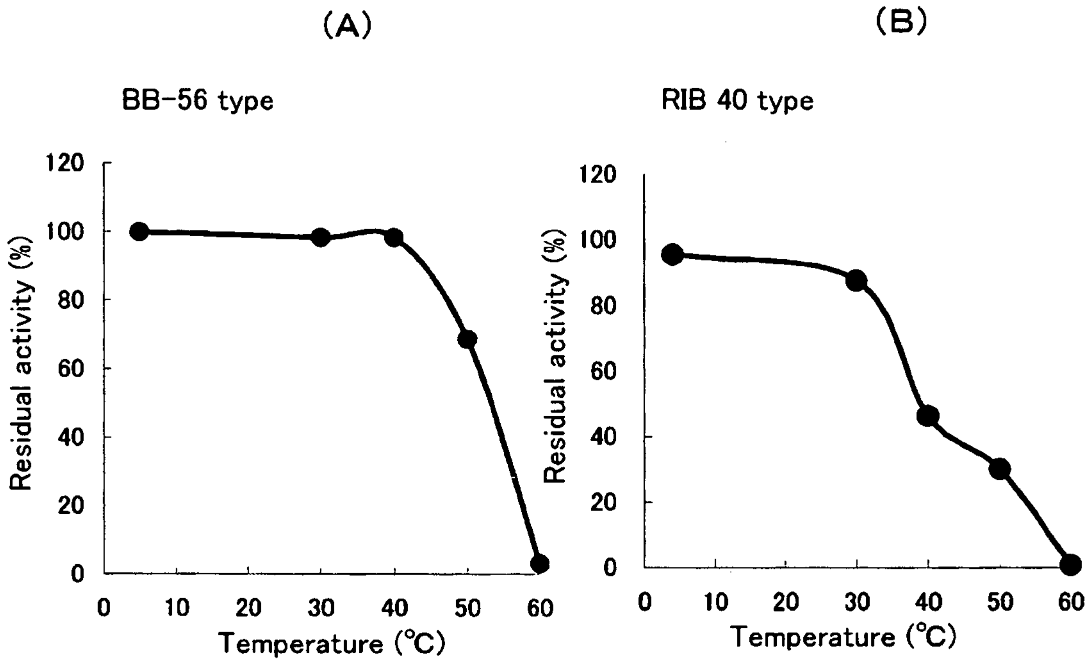
(A)
BB-56 type
Residual activity (%)
120
100
80
60
40
20
0
0
10
20
30
40
50
60
Temperature (°C)
(B)
RIB 40 type
Residual activity (%)
120
100
80
60
40
20
0
0
10
20
30
40
50
60
Temperature (°C)

*Fig. 11*

| Substrate | Aspergillus oryzae BB-56-derived FAD−GDH | Aspergillus oryzae RIB40-derived FAD−GDH |
|---|---|---|
| Glucose | 100 | 100 |
| Maltose | 0. 582 | 18. 1 |
| Galactose | 0. 349 | 5. 50 |
| Xylose | 22. 7 | 23. 6 |
| Maltotriose | 0. 239 | 0. 787 |
| Maltohexaose | 0. 479 | 7. 87 |
| Maltopentaose | 4. 55 | 7. 87 |

*Fig. 12*

| Supernatant | activity (u／ml) |
|---|---|
| BB－56 derived FAD－GDH | 2.06 |
| RIB40 derived FAD－GDH | ND |

| Inside of fungus body | activity (u／ml) |
|---|---|
| BB－56 derived FAD－GDH | 0.75 |
| RIB40 derived FAD－GDH | ND |

ND; No Detectable

*Fig. 13*

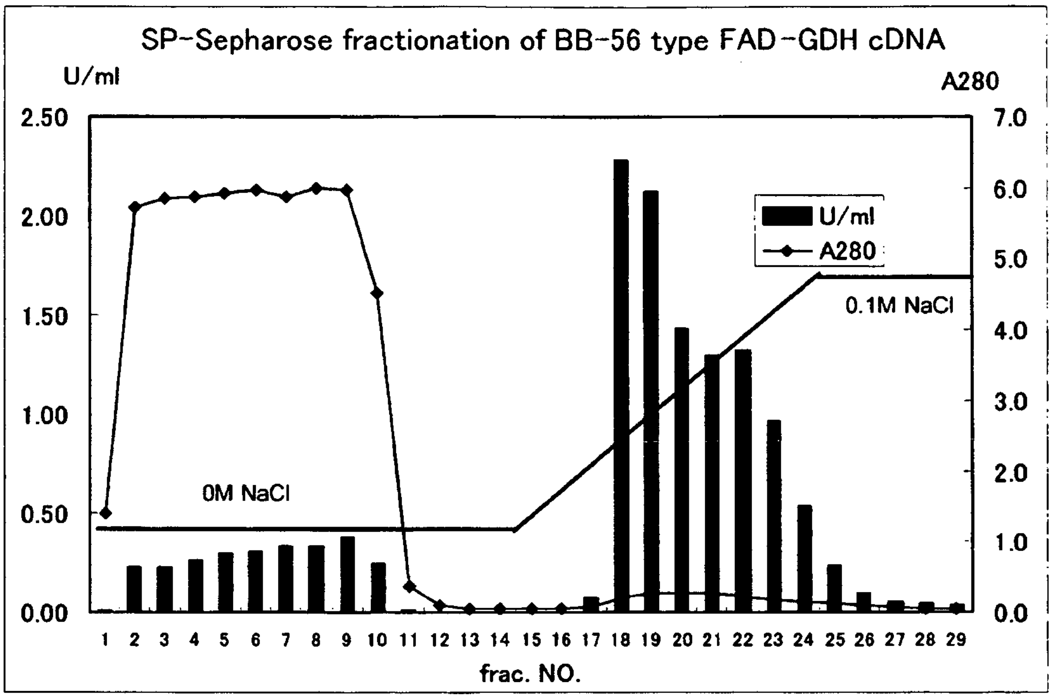
SP-Sepharose fractionation of BB-56 type FAD-GDH cDNA
U/ml
A280
2.50
2.00
1.50
1.00
0.50
0.00
7.0
6.0
5.0
4.0
3.0
2.0
1.0
0.0
U/ml
A280
0.1M NaCl
0M NaCl
1 2 3 4 5 6 7 8 9 10 11 12 13 14 15 16 17 18 19 20 21 22 23 24 25 26 27 28 29
frac. NO.

*Fig. 14*

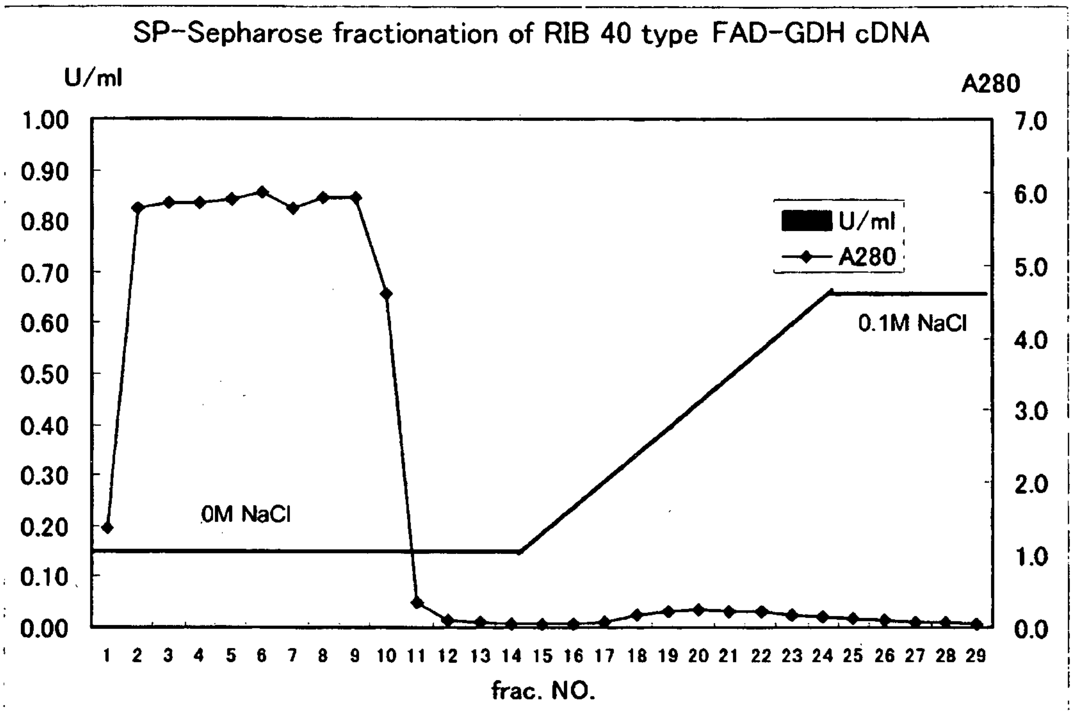
SP-Sepharose fractionation of RIB 40 type FAD-GDH cDNA
U/ml
A280
1.00
0.90
0.80
0.70
0.60
0.50
0.40
0.30
0.20
0.10
0.00
7.0
6.0
5.0
4.0
3.0
2.0
1.0
0.0
U/ml
A280
0.1M NaCl
0M NaCl
1 2 3 4 5 6 7 8 9 10 11 12 13 14 15 16 17 18 19 20 21 22 23 24 25 26 27 28 29
frac. NO.

*Fig. 15*

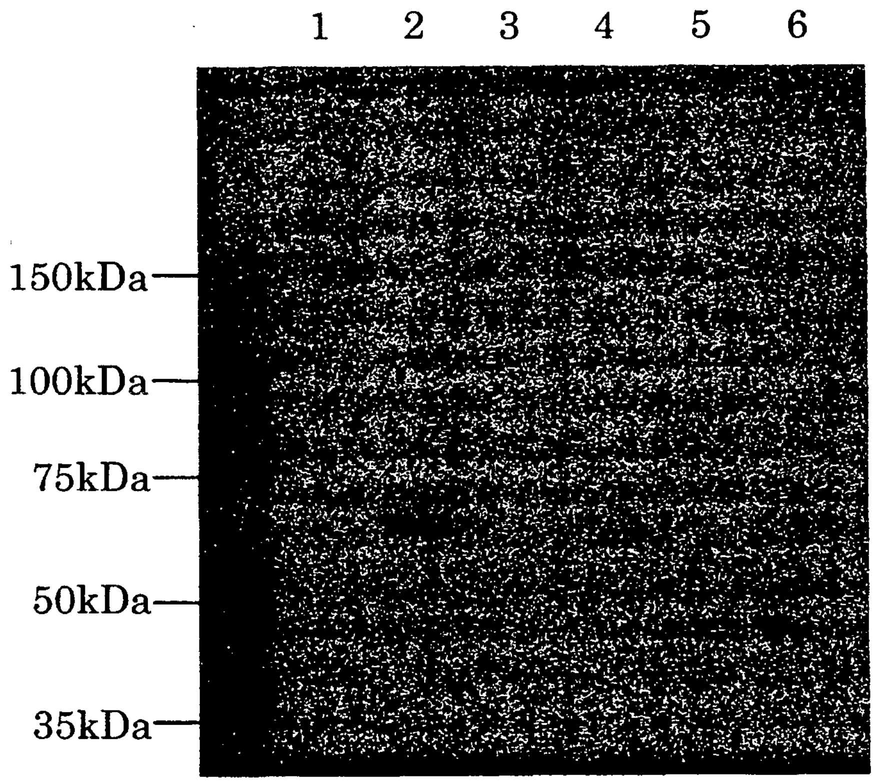


*Fig. 16*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000350588 A
- JP 2001197888 A
- JP 2001346587 A
- WO 2004058958 A
- EP 2022850 A
- WO 2007116710 A
- JP 2007060694 W
- EP 2022850 A1
- JP 2005176602 A
- JP 2003319786 A
- JP P2007308072 B


### Non-patent literature cited in the description


- **T.C. BAK ; R. SATO.** Studies on the glucose dehydrogenase of Aspergillus oryzae. I. Induction of its synthesis by p-benzoquinone and hydroquinone. *Biochim. Biophys. Acta,* 1967, vol. 139, 265-276
- **T.C. BAK.** Studies on the glucose dehydrogenase of Aspergillus oryzae. II. Purification and physical and chemical properties. *Biochim. Biophys. Acta,* 1967, vol. 139, 277-293
- **T.C. BAK.** Studies on the glucose dehydrogenase of Aspergillus oryzae. III. General enzymatic properties. *Biochim. Biophys. Acta,* 1967, vol. 146, 317-327
- **T.C. BAK ; R. SATO.** Studies on the glucose dehydrogenase of Aspergillus oryzae. IV. Histidyl residue as an active site. *Biochim. Biophys. Acta,* 1967, vol. 146, 328-335
- Molecular Cloning. Cold Spring Harbor Laboratory Press
- **POTTER, H. et al.** *Proc. Natl. Acad. Sci. U.S.A.,* 1984, vol. 81, 7161-7165
- **FELGNER, P.L. et al.** *Proc. Natl. Acad. Sci. U.S.A.,* 1984, vol. 84, 7413-7417
- **GRAESSMANN, M. ; GRAESSMANN, A.** *Proc. Natl. Acad. Sci. U.S.A.,* 1976, vol. 73, 366-370
- **HANAHAN, D.** *J. Mol. Biol.,* 1983, vol. 166, 557-580
- **SCHIESTL, R.H. et al.** *Curr. Genet.,* 1989, vol. 16, 339-346
- **YELTON, M.M et al.** *Proc. Natl. Acad. Sci.,* 1984, vol. 81, 1470-1474
- **BELSER et al.** *Methods in Enzymology,* vol. 51, 135

SEQUENCE LISTING

<110> AMANO ENZYME INC. YUUKI, Kensuke NAKANISHI, Yuji

<120> TRANSFORMANT COMPRISING FLAVIN ADENINE DINUCLEOTIDE-BINDING GLUCOSE DEHYDROGENASE GENE, METHOD OF PRODUCING FLAVIN ADENINE DINUCLEOTIDE-BINDING GLUCOSE DEHYDROGENASE USING THE SAME

<130> P07118P

<150> JP P2007-308072
<151> 2007-11-28

<160> 34

<170> PatentIn version 3.5

<210> 1
<211> 588
<212> PRT
<213> Aspergillus oryzae

<400> 1

```
Met Leu Phe Ser Leu Ala Phe Leu Ser Ala Leu Ser Leu Ala Thr Ala
1               5                   10                  15

Ser Pro Ala Gly Arg Ala Lys Asn Thr Thr Thr Tyr Asp Tyr Ile Val
            20                  25                  30

Val Gly Gly Gly Thr Ser Gly Leu Val Val Ala Asn Arg Leu Ser Glu
        35                  40                  45

Asn Pro Asp Val Ser Val Leu Leu Leu Glu Ala Gly Ala Ser Val Phe
    50                  55                  60

Asn Asn Pro Asp Val Thr Asn Ala Asn Gly Tyr Gly Leu Ala Phe Gly
65                  70                  75                  80

Ser Ala Ile Asp Trp Gln Tyr Gln Ser Ile Asn Gln Ser Tyr Ala Gly
                85                  90                  95

Gly Lys Gln Gln Val Leu Arg Ala Gly Lys Ala Leu Gly Gly Thr Ser
            100                 105                 110

Thr Ile Asn Gly Met Ala Tyr Thr Arg Ala Glu Asp Val Gln Ile Asp
        115                 120                 125

Val Trp Gln Lys Leu Gly Asn Glu Gly Trp Thr Trp Lys Asp Leu Leu
    130                 135                 140
```

```
Pro Tyr Tyr Leu Lys Ser Glu Asn Leu Thr Ala Pro Thr Ser Ser Gln
145                 150                 155                 160

Val Ala Ala Gly Ala Ala Tyr Asn Pro Ala Val Asn Gly Lys Glu Gly
                165                 170                 175

Pro Leu Lys Val Gly Trp Ser Gly Ser Leu Ala Ser Gly Asn Leu Ser
            180                 185                 190

Val Ala Leu Asn Arg Thr Phe Gln Ala Met Glu Asp Val Asn Gly Gly
        195                 200                 205

Lys Met Arg Gly Phe Asn Ile Tyr Pro Ser Thr Leu Asp Val Asp Leu
    210                 215                 220

Asn Val Arg Glu Asp Ala Ala Arg Ala Tyr Tyr Phe Pro Tyr Asp Asp
225                 230                 235                 240

Arg Lys Asn Leu His Leu Leu Glu Asn Thr Thr Ala Asn Arg Leu Phe
                245                 250                 255

Trp Lys Asn Gly Ser Ala Glu Glu Ala Ile Ala Asp Gly Val Glu Ile
            260                 265                 270

Thr Ser Ala Asp Gly Lys Val Thr Arg Val His Ala Lys Lys Glu Val
        275                 280                 285

Ile Ile Ser Ala Gly Ala Leu Arg Ser Pro Leu Ile Leu Glu Leu Ser
    290                 295                 300

Gly Val Gly Asn Pro Thr Ile Leu Lys Lys Asn Asn Ile Thr Pro Arg
305                 310                 315                 320

Val Asp Leu Pro Thr Val Gly Glu Asn Leu Gln Asp Gln Phe Asn Asn
                325                 330                 335

Gly Met Ala Gly Glu Gly Tyr Gly Val Leu Ala Gly Ala Ser Thr Val
            340                 345                 350

Thr Tyr Pro Ser Ile Ser Asp Val Phe Gly Asn Glu Thr Asp Ser Ile
        355                 360                 365

Val Ala Ser Leu Arg Ser Gln Leu Ser Asp Tyr Ala Ala Ala Thr Val
    370                 375                 380
```

```
Lys Val Ser Asn Gly His Met Lys Gln Glu Asp Leu Glu Arg Leu Tyr
385                 390                 395                 400

Gln Leu Gln Phe Asp Leu Ile Val Lys Asp Lys Val Pro Ile Ala Glu
                405                 410                 415

Ile Leu Phe His Pro Gly Gly Gly Asn Ala Val Ser Ser Glu Phe Trp
            420                 425                 430

Gly Leu Leu Pro Phe Ala Arg Gly Asn Ile His Ile Ser Ser Asn Asp
        435                 440                 445

Pro Thr Ala Pro Ala Ala Ile Asn Pro Asn Tyr Phe Met Phe Glu Trp
    450                 455                 460

Asp Gly Lys Ser Gln Ala Gly Ile Ala Lys Tyr Ile Arg Lys Ile Leu
465                 470                 475                 480

Arg Ser Ala Pro Leu Asn Lys Leu Ile Ala Lys Glu Thr Lys Pro Gly
                485                 490                 495

Leu Ser Glu Ile Pro Ala Thr Ala Ala Asp Glu Lys Trp Val Glu Trp
            500                 505                 510

Leu Lys Ala Asn Tyr Arg Ser Asn Phe His Pro Val Gly Thr Ala Ala
        515                 520                 525

Met Met Pro Arg Ser Ile Gly Gly Val Val Asp Asn Arg Leu Arg Val
    530                 535                 540

Tyr Gly Thr Ser Asn Val Arg Val Val Asp Ala Ser Val Leu Pro Phe
545                 550                 555                 560

Gln Val Cys Gly His Leu Val Ser Thr Leu Tyr Ala Val Ala Glu Arg
                565                 570                 575

Ala Ser Asp Leu Ile Lys Glu Asp Ala Lys Ser Ala
            580                 585
```

<210> 2
<211> 3226
<212> DNA
<213> Aspergillus oryzae

<400> 2

```
taggttttgt cacagccagg gtccgcgaaa accttactag cataaatcaa ccaccaaaca      60
cagtattcgg accgggtcta gttgcgatat cttcgttaac ggaatctgga ctctggtgta     120
```

```
atttcggagt ggacgtcggg agaatgatcc ttgggcttca tagcgcggcg atcaggtcaa   180
tgaggcaatt ttaggcctat tggagacttc ggatcctaga cattctgccg agccactttg   240
cctagactat tcctgacggg tcgggacttc accggtgaat gaattactcg gtatgccgat   300
tctgaataat accttaataa tgggaatttg atccactttt gggttcttcg ctaccacaga   360
gatgcgcact aaagattatt tgggcttagc tcgaatgtcc ctaacagtag cgtgtattgc   420
atcgtgctcg ctatagattg cagaaccata atctgagttt gagaacgtgt caactctcct   480
attaatcaat gaaagggaaa ggatgtgata tctaggagtg gaagattgag ctagcataga   540
taatatgggt ttaatcttcg tatgagatag agtaatagac ctttcgctgc tagagagcat   600
accacgcaaa gataatgcaa cgaagggacc ctcaactcga tcttatcaga tcagaaacgt   660
ccaccagttg cggcagaatt ccgcacagca gtattcgatt gatggaccca ctagcatgag   720
ctgataaatc catatctgct gaaacattag cttgcttaga agttgcttat ggatcattag   780
cttcattaac tggaggcagg atttgcagag aacttcacta tgatgcaatt caatcaccgt   840
gccgatagta cccagcagta aggtgaacgg aacccttcga attagactaa aatcaaaaaa   900
tatcatgcag tcatggactg catatataat ggttgatgcc agcctttttg cgaaaggatc   960
ttcttcaagg cggacagaga tcgacgcttg acatccaaac atgctcttct cactggcatt  1020
cctgagtgcc ctgtcgctgg ccacggcatc accggctgga cgggccaaga acactacgac  1080
atacgactac atcgttgtgg gaggcggcac aagtggtctt gtggtcgcaa atcgcctttc  1140
tgagaacccc gatgtctccg ttcttctgct tgaggccggt gcttctgtgt tcaacaaccc  1200
ggacgtaacc aacgctaacg gttatggatt ggcctttggc tcggccatcg actggcagta  1260
ccagtctatt aaccaaagct atgcaggagg taaacagcaa gttctgcgtg ctggtaaggc  1320
ccttggagga accagtacaa tcaatggtat gcttctatgg atgatctctt agtcggcatg  1380
gaccactgac gaccacagga atggcctata cccgcgcaga ggatgtccag attgacgttt  1440
ggcagaaact tggaaacgaa ggttggacgt ggaaagatct cctaccatac tacctgaaga  1500
gtgaaaactt gacggcccct accagctctc aggttgctgc tggcgctgct tataaccctg  1560
ccgtgaatgg aaaagaaggt cctctcaagg tcggctggtc gggaagcctg gcctccggta  1620
atctgtcagt tgctctgaac cgtacgttcc aagccatgga ggatgtcaat ggaggcaaga  1680
tgcgtggctt caacatctac ccatccaccc tcgacgttga cctcaatgtc cgcgaagatg  1740
cagcccgggc atactacttc ccttatgatg acaggaagaa ccttcacctg ctggagaaca  1800
ccactgccaa ccgccttttc tggaagaacg gctctgctga ggaagctatt gcggatggtg  1860
tcgagatcac ctccgctgat ggcaaggtca ctcgtgtgca tgcaaagaaa gaggtcatca  1920
```

```
tctctgctgg tgccctgcgg tctcctctca ttctcgagct ttcaggagtt ggaaacccaa      1980
cgtaagtgtt ccactgatgc cagcccctct ctatcaccgt ctctgaccct cgtagcatcc      2040
tcaaaaagaa caacataacc ccacgtgtcg atctccccac cgttggggag aacctccaag      2100
accagttcaa caacggcatg gctggcgaag gatacggcgt ccttgccggt gcctcaaccg      2160
tgacctaccc ttccatctcc gacgtcttcg gtaacgagac tgactctatc gttgcatctc      2220
tccgatctca actctccgac tacgccgccg cgaccgtcaa ggtcagcaac ggccacatga      2280
agcaggagga ccttgagcgc ctctaccagc tccaatttga cctcatcgtc aaggacaagg      2340
tccctatcgc cgagatcctc ttccaccccg gtggtggaaa cgccgtgtcc tccgaattct      2400
ggggcttgct tcccttcgcc cgtggcaaca tccacattag ctccaatgac ccgactgctc      2460
ccgccgccat caaccctaac tactttatgt tcgaatggga cggcaagagc caggccggta      2520
tcgccaagta catcaggaag attctccgca gcgcaccatt gaacaaactt attgcgaagg      2580
aaaccaagcc cggtctctct gagattccgg ccactgctgc ggatgagaag tgggttgaat      2640
ggctcaaggc taactgtaag ttgaatcctt tcttggcttc gatggtgagt ctgacgtgag      2700
ctctctagat cgttccaact tccaccccgt cggaactgct gccatgatgc ctcgttccat      2760
tggtggcgtt gttgataacc gtctccgggt ctatggtacc agcaatgttc gcgtcgtaga      2820
tgcgtctgtc ctgcccttcc aggtttgcgg ccacttggtt agcacgcttt atgccgttgc      2880
cgagcgcgct tccgacttga ttaaggagga tgcgaagagt gcttagtttg gtagataaag      2940
ctgcattatc gtagggacag tgtcccgtac caattcgtgc accgtatgaa gagagaaagt      3000
ggtttcagaa ttgggttgtc aaatgtggat ttctgtttca ttgtttaatg tattattagt      3060
ctggattttg tggctgtttg aatgcagtga ctcttggtta cataatttga tacggggtat      3120
gttcgcttgt tcagtcatcg cacgtgtaat gtatggcata cctaggttat tggtaactca      3180
tattacaaaa ttgaagagaa cgagagatcg aagtatttcc catggc                     3226
```

<210> 3
<211> 25
<212> DNA
<213> Artificial

<220>
<223> primer FG-F

<400> 3
taggttttgt cacagccagg gtccg 25

<210> 4
<211> 25
<212> DNA
<213> Artificial

<220>
<223> primer FG-R

<400> 4
gccatgggaa atacttcgat ctctc 25

<210> 5
<211> 18
<212> DNA
<213> Artificial

<220>
<223> primer FG-1

<400> 5
agtgaaaact tgacggcc 18

<210> 6
<211> 18
<212> DNA
<213> Artificial

<220>
<223> primer FG-2

<400> 6
ttcaggagtt ggaaaccc 18

<210> 7
<211> 18
<212> DNA
<213> Artificial

<220>
<223> primer FG-3

<400> 7
tatgttcgaa tgggacgg 18

<210> 8
<211> 18
<212> DNA
<213> Artificial

<220>
<223> primer FG-4

<400> 8
agaacgtgtc aactctcc 18

<210> 9
<211> 18
<212> DNA
<213> Artificial

<220>
<223> primer FG-5

<400> 9
tatgcatttc tcactggc 18

<210> 10
<211> 18
<212> DNA
<213> Artificial

<220>
<223> primer FG-6

<400> 10
gattgtactg gttcctcc 18

<210> 11
<211> 18
<212> DNA
<213> Artificial

<220>
<223> primer FG-7

<400> 11
tgcaccgtat gaagagag 18

<210> 12
<211> 18
<212> DNA
<213> Artificial

<220>
<223> primer FG-8

<400> 12
ttgctggtac catagacc 18

<210> 13
<211> 18
<212> DNA
<213> Artificial

<220>
<223> primer FG-9

<400> 13
ttgctgacct tgacggtc 18

<210> 14
<211> 18
<212> DNA
<213> Artificial

<220>
<223> primer FG-10

<400> 14
attgaggtca acgtcgag 18

<210> 15
<211> 18
<212> DNA
<213> Artificial

<220>
<223> primer FG-11

<400> 15
gcacggtgat tgaattgc 18

<210> 16
<211> 18
<212> DNA
<213> Artificial

<220>
<223> primer FG-12

<400> 16
tctgtggtag cgaagaac 18

<210> 17
<211> 18
<212> DNA
<213> Artificial ,

<220>
<223> primer M13-M5

<400> 17
cgaaaggggg atgtgctg 18

<210> 18
<211> 18
<212> DNA
<213> Artificial

<220>
<223> primer M13-RV2

<400> 18
cttccggctc gtatgttg 18

<210> 19
<211> 3241
<212> DNA
<213> Aspergillus oryzae

<400> 19

taggttttgt cacagccagg gtccgcgaaa accttactag cataaatcaa ccaccaaaca 60

```
cagtattcgg accgggtcta gttgcgacat cttcgttaac ggaatctgga ctctggtgta      120
atttcggagt ggacgtcggg agaatgatcc ttgggtttca tagcgcggcg atcaggtcaa      180
tgaggcaatt ttaggcctat tggagacttc ggatcctaga cattctgccg agccactttg      240
cctagactat tcctgacggg tcgggacttc accggtgaat gaattactcg gtatgccgat      300
tctgaataat accttaataa tgggaatttg atccactttt gggttcttcg ctaccacaga      360
gatgcgcact aaagattatt tgggcttagc tcgaatgtcc ctaacagtag cgtgtattgc      420
atcgtgctcg ctatagattg cagaaccata atctgagttt gagaacgtgt caactctcct      480
attaatcaat gaaagggaaa ggatgtgata tctaggagtg gaagattgag ctagcataga      540
taatatgggt ttaatcttcg tatgagatag agtaatagac ctttcgctgc tagagagcat      600
accacgcaaa gataatgcaa cgaagggacc ctcaactcga tcttatcaga tcagaaacgt      660
ccaccagttg cggcagaatt ccgcacagca gtattcgatt gatggaccca ctagcatgag      720
ctgataaatc catatctgct gaaacattag cttgcttaga agttgcttat ggatcattag      780
cttcattaac tggaggcagg atttgcagag aacttcacta tgatgcaatt caatcaccgt      840
gccgatagta cccagcagta aggtgaacgg aacccttcga attagactaa aatcaaaaaa      900
tatcatgcag tcatggactg catatataat ggttgatgcc agccttttttg cgaaaggatc      960
ttcttcaagg cggacagaga tcgacgcttg acatccaaac atgctcttct cactggcatt     1020
cctgagtgcc ctgtcgctgg ccacggcatc accggctgga cgggccaaga acactacgac     1080
atacgactac atcgttgtgg gaggcggcac aagtggtctt gtggtcgcaa atcgcctttc     1140
tgagaacccc gatgtctccg ttcttctgct tgaggccggt gcttctgtgt tcaacaaccc     1200
ggacgtaacc aacgctaacg gttatggatt ggcctttggc tcggccatcg actggcagta     1260
ccagtctatt aaccaaagct atgcaggagg taaacagcaa gttctgcgtg ctggtaaggc     1320
ccttggagga accagtacaa tcaatggtat gcttctatgg atgatctctt agtcggcatg     1380
gaccactgac gaccacagga atggcctata cccgcgcaga ggatgtccag attgacgttt     1440
ggcagaaact tggaaacgaa ggttggacgt ggaaagatct cctaccatac tacctgaaga     1500
gtgaaaactt gacggcccct accagctctc aggttgctgc tggcgctgct tataaccctg     1560
ccgtgaatgg aaaagaaggt cctctcaagg tcggctggtc gggaagcctg gcctccggta     1620
atctgtcagt tgctctgaac cgtacgttcc aagccgctgg tgttccatgg gttgaggatg     1680
tcaatggagg caagatgcgt ggcttcaaca tctacccatc caccctcgac gttgacctca     1740
atgtccgcga agatgcagcc cgggcatact acttccctta tgatgacagg aagaaccttc     1800
acctgctgga gaacaccact gccaaccgcc ttttctggaa gaacggctct gctgaggaag     1860
```

ctattgcgga tggtgtcgag atcacctccg ctgatggcaa ggtcactcgt gtgcatgcaa 1920

agaaagaggt catcatctct gctggtgccc tgcggtctcc tctcattctc gagctttcag 1980

gagttggaaa cccaacgtaa gtgttccact gatgccagcc cctctctatc accgtctctg 2040

accctcgtag catcctcaaa aagaacaaca taaccccacg tgtcgatctc cccaccgttg 2100

gggagaacct ccaagaccag ttcaacaacg gcatggctgg cgaaggatac ggcgtccttg 2160

ccggtgcctc aaccgtgacc tacccttcca tctccgacgt cttcggtaac gagactgact 2220

ctatcgttgc atctctccga tctcaactct ccgactacgc cgccgcgacc gtcaaggtca 2280

gcaacggcca catgaagcag gaggaccttg agcgcctcta ccagctccaa tttgacctca 2340

tcgtcaagga caaggtccct atcgccgaga tcctcttcca ccccggtggt ggaaacgccg 2400

tgtcctccga attctggggc ttgcttccct tcgcccgtgg caacatccac attagctcca 2460

atgacccgac tgctcccgcc gccatcaacc ctaactactt tatgttcgaa tgggacggca 2520

agagccaggc cggtatcgcc aagtacatca ggaagattct ccgcagcgca ccattgaaca 2580

aacttattgc gaaggaaacc aagcccggtc tctctgagat tccggccact gctgcggatg 2640

agaagtgggt tgaatggctc aaggctaact gtaagttgaa tcctttcttg gcttcgatgg 2700

tgagtctgac gtgagctctc tagatcgttc caacttccac cccgtcggaa ctgctgccat 2760

gatgcctcgt tccattggtg gcgttgttga taaccgtctc cgggtctatg gtaccagcaa 2820

tgttcgcgtc gtagatgcgt ctgtcctgcc cttccaggtt tgcggccact tggttagcac 2880

gctttatgcc gttgccgagc gcgcttccga cttgattaag gaggatgcga agagtgctta 2940

gtttggtaga taaagctgca ttatcgtagg gacagtgtcc cgtaccaatt cgtgcaccgt 3000

atgaagagag aaagtggttt cagaattggg ttgtcaaatg tggatttctg tttcattgtt 3060

taatgtatta ttagtctgga ttttgtggct gtttgaatgc agtgactctt ggttacataa 3120

tttgatacgg ggtatgttcg cttgttcagt catcgcacgt gtaatgtatg gcatacctag 3180

gttattggta actcatatta caaaattgaa gagaacgaga gatcgaagta tttcccatgg 3240

c 3241

<210> 20
<211> 593
<212> PRT
<213> Aspergillus oryzae

<400> 20

Met Leu Phe Ser Leu Ala Phe Leu Ser Ala Leu Ser Leu Ala Thr Ala
1               5                   10                  15

Ser Pro Ala Gly Arg Ala Lys Asn Thr Thr Thr Tyr Asp Tyr Ile Val

```
            20                  25                  30

Val Gly Gly Gly Thr Ser Gly Leu Val Val Ala Asn Arg Leu Ser Glu
        35                  40                  45

Asn Pro Asp Val Ser Val Leu Leu Leu Glu Ala Gly Ala Ser Val Phe
    50                  55                  60

Asn Asn Pro Asp Val Thr Asn Ala Asn Gly Tyr Gly Leu Ala Phe Gly
65                  70                  75                  80

Ser Ala Ile Asp Trp Gln Tyr Gln Ser Ile Asn Gln Ser Tyr Ala Gly
                85                  90                  95

Gly Lys Gln Gln Val Leu Arg Ala Gly Lys Ala Leu Gly Gly Thr Ser
            100                 105                 110

Thr Ile Asn Gly Met Ala Tyr Thr Arg Ala Glu Asp Val Gln Ile Asp
        115                 120                 125

Val Trp Gln Lys Leu Gly Asn Glu Gly Trp Thr Trp Lys Asp Leu Leu
    130                 135                 140

Pro Tyr Tyr Leu Lys Ser Glu Asn Leu Thr Ala Pro Thr Ser Ser Gln
145                 150                 155                 160

Val Ala Ala Gly Ala Ala Tyr Asn Pro Ala Val Asn Gly Lys Glu Gly
                165                 170                 175

Pro Leu Lys Val Gly Trp Ser Gly Ser Leu Ala Ser Gly Asn Leu Ser
            180                 185                 190

Val Ala Leu Asn Arg Thr Phe Gln Ala Ala Gly Val Pro Trp Val Glu
        195                 200                 205

Asp Val Asn Gly Gly Lys Met Arg Gly Phe Asn Ile Tyr Pro Ser Thr
    210                 215                 220

Leu Asp Val Asp Leu Asn Val Arg Glu Asp Ala Ala Arg Ala Tyr Tyr
225                 230                 235                 240

Phe Pro Tyr Asp Asp Arg Lys Asn Leu His Leu Leu Glu Asn Thr Thr
                245                 250                 255

Ala Asn Arg Leu Phe Trp Lys Asn Gly Ser Ala Glu Glu Ala Ile Ala
            260                 265                 270
```

```
Asp Gly Val Glu Ile Thr Ser Ala Asp Gly Lys Val Thr Arg Val His
        275                 280                 285

Ala Lys Lys Glu Val Ile Ile Ser Ala Gly Ala Leu Arg Ser Pro Leu
    290                 295                 300

Ile Leu Glu Leu Ser Gly Val Gly Asn Pro Thr Ile Leu Lys Lys Asn
305                 310                 315                 320

Asn Ile Thr Pro Arg Val Asp Leu Pro Thr Val Gly Glu Asn Leu Gln
                325                 330                 335

Asp Gln Phe Asn Asn Gly Met Ala Gly Glu Gly Tyr Gly Val Leu Ala
            340                 345                 350

Gly Ala Ser Thr Val Thr Tyr Pro Ser Ile Ser Asp Val Phe Gly Asn
        355                 360                 365

Glu Thr Asp Ser Ile Val Ala Ser Leu Arg Ser Gln Leu Ser Asp Tyr
    370                 375                 380

Ala Ala Ala Thr Val Lys Val Ser Asn Gly His Met Lys Gln Glu Asp
385                 390                 395                 400

Leu Glu Arg Leu Tyr Gln Leu Gln Phe Asp Leu Ile Val Lys Asp Lys
                405                 410                 415

Val Pro Ile Ala Glu Ile Leu Phe His Pro Gly Gly Gly Asn Ala Val
            420                 425                 430

Ser Ser Glu Phe Trp Gly Leu Leu Pro Phe Ala Arg Gly Asn Ile His
        435                 440                 445

Ile Ser Ser Asn Asp Pro Thr Ala Pro Ala Ala Ile Asn Pro Asn Tyr
    450                 455                 460

Phe Met Phe Glu Trp Asp Gly Lys Ser Gln Ala Gly Ile Ala Lys Tyr
465                 470                 475                 480

Ile Arg Lys Ile Leu Arg Ser Ala Pro Leu Asn Lys Leu Ile Ala Lys
                485                 490                 495

Glu Thr Lys Pro Gly Leu Ser Glu Ile Pro Ala Thr Ala Ala Asp Glu
            500                 505                 510
```

```
Lys Trp Val Glu Trp Leu Lys Ala Asn Tyr Arg Ser Asn Phe His Pro
        515                 520                 525

Val Gly Thr Ala Ala Met Met Pro Arg Ser Ile Gly Gly Val Val Asp
    530                 535                 540

Asn Arg Leu Arg Val Tyr Gly Thr Ser Asn Val Arg Val Val Asp Ala
545                 550                 555                 560

Ser Val Leu Pro Phe Gln Val Cys Gly His Leu Val Ser Thr Leu Tyr
                565                 570                 575

Ala Val Ala Glu Arg Ala Ser Asp Leu Ile Lys Glu Asp Ala Lys Ser
            580                 585                 590

Ala
```

<210> 21
<211> 33
<212> DNA
<213> Artificial

<220>
<223> primer

<400> 21
tacgcgtcga cccaagccgc tgctggaatt gac 33

<210> 22
<211> 37
<212> DNA
<213> Artificial

<220>
<223> primer

<400> 22
aagggaagct tgatcaatac cgtacgggag attgtac 37

<210> 23
<211> 24
<212> DNA
<213> Artificial

<220>
<223> primer

<400> 23
ccaaacatgc atttctcact ggca 24

<210> 24
<211> 36
<212> DNA

<213> Artificial

<220>
<223> primer

<400> 24
tacgcgtcga cgccatggga aatacttcga tctctc 36

<210> 25
<211> 28
<212> DNA
<213> Artificial

<220>
<223> primer

<400> 25
ggggtaccga attcatggtg ttttgatc 28

<210> 26
<211> 30
<212> DNA
<213> Artificial

<220>
<223> primer

<400> 26
gagaccacca cgcgacatgc ataaatgcct 30

<210> 27
<211> 25
<212> DNA
<213> Artificial

<220>
<223> primer

<400> 27
tatgaccaag cttacgaatt catgg 25

<210> 28
<211> 34
<212> DNA
<213> Artificial

<220>
<223> primer

<400> 28
aagggaagct tgccatggga aatacttcga tctc 34

<210> 29
<211> 29
<212> DNA
<213> Artificial

<220>

<223> primer

<400> 29
ataagcttaa ccatggtctt ctcactggc 29

<210> 30
<211> 30
<212> DNA
<213> Artificial

<220>
<223> primer

<400> 30
atggatcctt actaagcact cttcgcatcc 30

<210> 31
<211> 18
<212> DNA
<213> Aspergillus oryzae

<400> 31
gctggtgttc catgggtt 18

<210> 32
<211> 6
<212> PRT
<213> Aspergillus oryzae

<400> 32

Ala Gly Val Pro Trp Val
1               5

<210> 33
<211> 1782
<212> DNA
<213> Aspergillus oryzae

<400> 33

atggtcttct cactggcatt cctgagtgcc ctgtcgctgg ccacggcatc accggctgga 60

cgggccaaga acactacgac atacgactac atcgttgtgg gaggcggcac aagtggtctt 120

gtggtcgcaa atcgcctttc tgagaacccc gatgtctccg ttcttctgct tgaggccggt 180

gcttctgtgt tcaacaaccc ggacgtaacc aacgctaacg gttatggatt ggcctttggc 240

tcggccatcg actggcagta ccagtctatt aaccaaagct atgcaggagg taaacagcaa 300

gttctgcgtg ctggtaaggc ccttggagga accagtacaa tcaatggaat ggcctatacc 360

```
cgcgcagagg atgtccagat tgacgtttgg cagaaacttg gaaacgaagg ttggacgtgg      420
aaagatctcc taccatacta cctgaagagt gaaaacttga cggcccctac cagctctcag      480
gttgctgctg gcgctgctta taaccctgcc gtgaatggaa aagaaggtcc tctcaaggtc      540
ggctggtcgg gaagcctggc ctccggtaat ctgtcagttg ctctgaaccg tacgttccaa      600
gccgctggtg ttccatgggt tgaggatgtc aatggaggca agatgcgtgg cttcaacatc      660
tacccatcca ccctcgacgt tgacctcaat gtccgcgaag atgcagcccg ggcatactac      720
ttcccttatg atgacaggaa gaaccttcac ctgctggaga acaccactgc caaccgcctt      780
ttctggaaga acggctctgc tgaggaagct attgcggatg gtgtcgagat cacctccgct      840
gatggcaagg tcactcgtgt gcatgcaaag aaagaggtca tcatctctgc tggtgccctg      900
cggtctcctc tcattctcga gctttcagga gttggaaacc caaccatcct caaaaagaac      960
aacataaccc cacgtgtcga tctccccacc gttggggaga acctccaaga ccagttcaac     1020
aacggcatgg ctggcgaagg atacggcgtc cttgccggtg cctcaaccgt gacctaccct     1080
tccatctccg acgtcttcgg taacgagact gactctatcg ttgcatctct ccgatctcaa     1140
ctctccgact acgccgccgc gaccgtcaag gtcagcaacg gccacatgaa gcaggaggac     1200
cttgagcgcc tctaccagct ccaatttgac ctcatcgtca aggacaaggt ccctatcgcc     1260
gagatcctct tccaccccgg tggtggaaac gccgtgtcct ccgaattctg ggcttgctt      1320
cccttcgccc gtggcaacat ccacattagc tccaatgacc cgactgctcc cgccgccatc     1380
aaccctaact actttatgtt cgaatgggac ggcaagagcc aggccggtat cgccaagtac     1440
atcaggaaga ttctccgcag cgcaccattg aacaaactta ttgcgaagga aaccaagccc     1500
ggtctctctg agattccggc cactgctgcg gatgagaagt gggttgaatg gctcaaggct     1560
aactatcgtt ccaacttcca ccccgtcgga actgctgcca tgatgcctcg ttccattggt     1620
ggcgttgttg ataaccgtct ccgggtctat ggtaccagca atgttcgcgt cgtagatgcg     1680
tctgtcctgc ccttccaggt ttgcggccac ttggttagca cgctttatgc cgttgccgag     1740
cgcgcttccg acttgattaa ggaggatgcg aagagtgctt ag                        1782
```

<210> 34
<211> 593
<212> PRT
<213> Aspergillus oryzae

<400> 34

```
Met Val Phe Ser Leu Ala Phe Leu Ser Ala Leu Ser Leu Ala Thr Ala
1               5                   10                  15

Ser Pro Ala Gly Arg Ala Lys Asn Thr Thr Thr Tyr Asp Tyr Ile Val
```

20 25 30

Val Gly Gly Gly Thr Ser Gly Leu Val Val Ala Asn Arg Leu Ser Glu
35 40 45

Asn Pro Asp Val Ser Val Leu Leu Leu Glu Ala Gly Ala Ser Val Phe
50 55 60

Asn Asn Pro Asp Val Thr Asn Ala Asn Gly Tyr Gly Leu Ala Phe Gly
65 70 75 80

Ser Ala Ile Asp Trp Gln Tyr Gln Ser Ile Asn Gln Ser Tyr Ala Gly
85 90 95

Gly Lys Gln Gln Val Leu Arg Ala Gly Lys Ala Leu Gly Gly Thr Ser
100 105 110

Thr Ile Asn Gly Met Ala Tyr Thr Arg Ala Glu Asp Val Gln Ile Asp
115 120 125

Val Trp Gln Lys Leu Gly Asn Glu Gly Trp Thr Trp Lys Asp Leu Leu
130 135 140

Pro Tyr Tyr Leu Lys Ser Glu Asn Leu Thr Ala Pro Thr Ser Ser Gln
145 150 155 160

Val Ala Ala Gly Ala Ala Tyr Asn Pro Ala Val Asn Gly Lys Glu Gly
165 170 175

Pro Leu Lys Val Gly Trp Ser Gly Ser Leu Ala Ser Gly Asn Leu Ser
180 185 190

Val Ala Leu Asn Arg Thr Phe Gln Ala Ala Gly Val Pro Trp Val Glu
195 200 205

Asp Val Asn Gly Gly Lys Met Arg Gly Phe Asn Ile Tyr Pro Ser Thr
210 215 220

Leu Asp Val Asp Leu Asn Val Arg Glu Asp Ala Ala Arg Ala Tyr Tyr
225 230 235 240

Phe Pro Tyr Asp Asp Arg Lys Asn Leu His Leu Leu Glu Asn Thr Thr
245 250 255

Ala Asn Arg Leu Phe Trp Lys Asn Gly Ser Ala Glu Glu Ala Ile Ala
260 265 270

Asp Gly Val Glu Ile Thr Ser Ala Asp Gly Lys Val Thr Arg Val His
275 280 285

Ala Lys Lys Glu Val Ile Ile Ser Ala Gly Ala Leu Arg Ser Pro Leu
290 295 300

Ile Leu Glu Leu Ser Gly Val Gly Asn Pro Thr Ile Leu Lys Lys Asn
305 310 315 320

Asn Ile Thr Pro Arg Val Asp Leu Pro Thr Val Gly Glu Asn Leu Gln
325 330 335

Asp Gln Phe Asn Asn Gly Met Ala Gly Glu Gly Tyr Gly Val Leu Ala
340 345 350

Gly Ala Ser Thr Val Thr Tyr Pro Ser Ile Ser Asp Val Phe Gly Asn
355 360 365

Glu Thr Asp Ser Ile Val Ala Ser Leu Arg Ser Gln Leu Ser Asp Tyr
370 375 380

Ala Ala Ala Thr Val Lys Val Ser Asn Gly His Met Lys Gln Glu Asp
385 390 395 400

Leu Glu Arg Leu Tyr Gln Leu Gln Phe Asp Leu Ile Val Lys Asp Lys
405 410 415

Val Pro Ile Ala Glu Ile Leu Phe His Pro Gly Gly Gly Asn Ala Val
420 425 430

Ser Ser Glu Phe Trp Gly Leu Leu Pro Phe Ala Arg Gly Asn Ile His
435 440 445

Ile Ser Ser Asn Asp Pro Thr Ala Pro Ala Ala Ile Asn Pro Asn Tyr
450 455 460

Phe Met Phe Glu Trp Asp Gly Lys Ser Gln Ala Gly Ile Ala Lys Tyr
465 470 475 480

Ile Arg Lys Ile Leu Arg Ser Ala Pro Leu Asn Lys Leu Ile Ala Lys
485 490 495

Glu Thr Lys Pro Gly Leu Ser Glu Ile Pro Ala Thr Ala Ala Asp Glu
500 505 510

```
Lys Trp Val Glu Trp Leu Lys Ala Asn Tyr Arg Ser Asn Phe His Pro
        515                 520                 525

Val Gly Thr Ala Ala Met Met Pro Arg Ser Ile Gly Gly Val Val Asp
    530                 535                 540

Asn Arg Leu Arg Val Tyr Gly Thr Ser Asn Val Arg Val Val Asp Ala
545                 550                 555                 560

Ser Val Leu Pro Phe Gln Val Cys Gly His Leu Val Ser Thr Leu Tyr
                565                 570                 575

Ala Val Ala Glu Arg Ala Ser Asp Leu Ile Lys Glu Asp Ala Lys Ser
            580                 585                 590

Ala
```